# EUROPEAN PATENT APPLICATION

(11) **EP 4 088 739 A1**
(43) Date of publication of application: **16.11.2022**
(21) Application number: 20905452.7
(22) Date of filing: 21.12.2020
(51) Int. Cl.: A61K 39/395, A61P 35/00, C07K 5/062, C07K 16/28, A61K 47/68, C12N 15/13, A61K 31/357

(54) **METHOD FOR PRODUCING ERIBULIN-BASED ANTIBODY-DRUG CONJUGATE**

(30) Priority: 23.12.2019 JP 2019231938
(71) Applicant: Eisai R&D Management Co., Ltd., Tokyo 112-8088 (JP)
(72) Inventor: MATSUO Kimihiro, Kamisu-shi, Ibaraki 314-0255 (JP); NAKAMURA Taiju, Kamisu-shi, Ibaraki 314-0255 (JP); MIYASHITA Yusuke, Kamisu-shi, Ibaraki 314-0255 (JP)
(74) Representative: Hoffmann Eitle
(86) International application number: PCT/JP2020/047706
(87) International publication number: WO 2021/132166

(57) **Abstract**

Provided is a method of producing an antibody-drug conjugate represented by Formula (I), 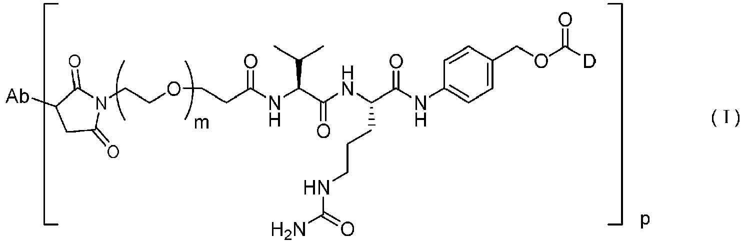 in the formula, Ab is an antibody or an antigen-binding fragment thereof, D is eribulin, m is an integer of 1 to 10, and p is an integer of 1 to 8,
the method comprising:
a step 1 of obtaining a compound represented by Formula (B) by reaction of eribulin or a salt thereof with a compound represented by Formula (A), 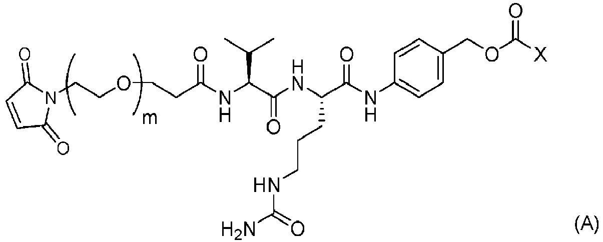 in the formula, m is an integer of 1 to 10 and X is a phenoxy group or a nitrophenoxy group, and 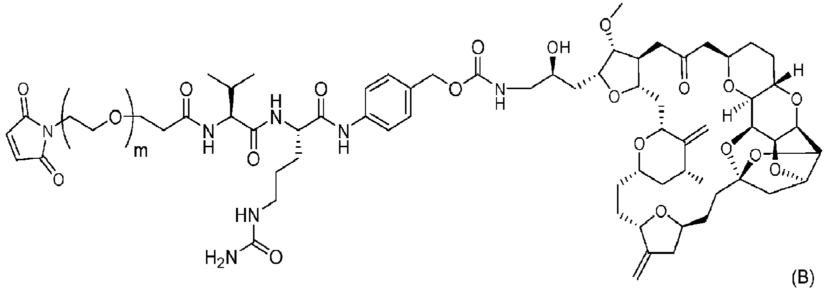 in the formula, m is an integer of 1 to 10, and
a step 2 of obtaining the antibody-drug conjugate represented by Formula (I) by reaction of the compound represented by Formula (B) with Ab.

## Description

### Technical Field

The present invention relates to a method of producing an eribulin based antibody-drug conjugate.

### Background Art

A cancer is one of the main reasons for disease and death, and about 14,000,000 new cancer cases and about 8,200,000 cancer deaths were reported in 2012. The most common reasons for cancer deaths are lung cancer (1,590,000 deaths), liver cancer (745,000 deaths), gastric cancer (723,000 deaths), colorectal cancer (694,000 deaths), breast cancer (521,000 deaths), and esophageal cancer (400,000 deaths). It is expected that the number of new cancer cases will increase by about 70% in the next 20 years and about 22,000,000 new cancer cases occur per year (Non-Patent Literature 1).

Microtubules are dynamic filamentous cytoskeletal proteins relating to various cell functions including intracellular migration and transport, cell signaling, and cell shape maintenance. Microtubules also play an important role in mitotic cell division by forming a mitotic spindle required to divide a chromosome into two daughter cells. Most of the biological functions of microtubules in all the cells are regulated by their polymerization dynamics, which occurs by the reversible, non-covalent addition of a- and β-tubulin dimers at both ends of microtubules. This dynamic behavior and resulting control over microtubule length is vital to the proper functioning of the spindle. Even minor alteration of microtubule dynamics can engage the spindle checkpoint, arrest cell cycle progression at mitosis, and subsequently lead to cell death (Non-Patent Literature 2). Due to their rapid cell division, cancer cells are generally more sensitive to compounds that bind to tubulin and disrupt its normal function, as compared to normal cells. For this reason, tubulin inhibitors and other microtubule-targeted agents have become a promising class of drugs for the treatment of cancer (Non-Patent Literature 3).

Folate receptor alpha (FRA) is a glycophosphatidylinositol (GPI)-linked membrane protein that binds folate. While the role of FRA in the biology of normal tissues and cancerous tissues is not fully understood, FRA is highly overexpressed on a high percentage of ovarian cancers of epithelial origin (Non-Patent Literature 4), as well as in a percentage of non-small cell lung cancer (Non-Patent Literature 5). FRA also has limited expression in normal tissues. These properties make FRA an attractive target for cancer immunotherapy.

The proto-oncogene human epidermal growth factor receptor 2 (HER2) encodes a transmembrane tyrosine kinase receptor that belongs to the human epidermal growth factor receptor (EGFR) family (Non-Patent Literature 6). Overexpression of HER2 enables constitutive activation of growth factor signaling pathways, such as the PI3K-AKT-mTOR pathway, and thereby serves as an oncogenic driver in several types of cancers, including approximately 20% of invasive breast cancers (Non-Patent Literatures 7 and 8). Since HER2 amplification mediates the transformed phenotype, HER2 is another promising target for cancer treatment.

### Citation List

### Patent Literature

[Patent Literature 1] WO2017/151979

### Non Patent Literature

[Non-Patent Literature 1] World Cancer Report 2014
[Non-Patent Literature 2] Mukhtar et al. (2014) Mol. Cancer Ther. 13:275-84
[Non-Patent Literature 3] Dumontet and Jordan (2010) Nat. Rev. Drug Discov. 9:790-803
[Non-Patent Literature 4] O'Shannessy et al. (2013) Int. J. Gynecol. Pathol. 32(3):258-68
[Non-Patent Literature 5] Christoph et al. (2014) Clin. Lung Cancer 15(5):320-30
[Non-Patent Literature 6] King et al. (1985) Science 229:974-6
[Non-Patent Literature 7] Slamon et al. (1989) Science 244:707-12
[Non-Patent Literature 8] Gajria and Chandarlapaty (2011) Expert Rev. Anticancer Ther. 11:263-75
[Non-Patent Literature 9] O'Shannessy et al., (2011) Oncotarget 2:1227-43

### Summary of Invention

### Technical Problem

An object of the present invention is to provide a method of producing an antibody-drug conjugate with high yield and a synthetic intermediate useful for the method.

### Solution to Problem

The present invention provides the following [1] to [8].
[1] A method of producing an antibody-drug conjugate represented by Formula (I), 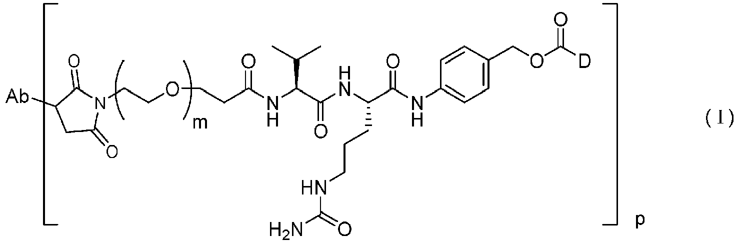
   in the formula, Ab is an antibody or an antigen-binding fragment thereof, D is eribulin, m is an integer of 1 to 10, and p is an integer of 1 to 8],
   the method including:
      a step 1 of obtaining a compound represented by Formula (B) by reaction of eribulin or a salt thereof with a compound represented by Formula (A), 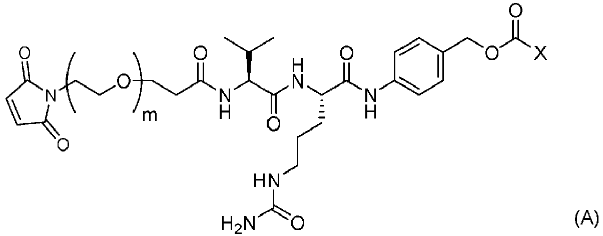 in the formula, m is an integer of 1 to 10 and X is a phenoxy group or a nitrophenoxy group, and 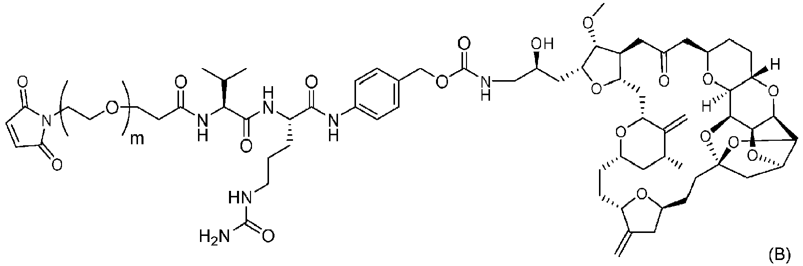 in the formula, m is an integer of 1 to 10, and
      a step 2 of obtaining the antibody-drug conjugate represented by Formula (I) by reaction of the compound represented by Formula (B) with Ab.
[2] The method according to [1], in which Ab includes (i) a heavy chain domain including an amino acid sequence represented by SEQ ID NO: 1 and a light chain domain including an amino acid sequence represented by SEQ ID NO: 6, (ii) a heavy chain variable domain including an amino acid sequence represented by SEQ ID NO: 23 and a light chain variable domain including an amino acid sequence represented by SEQ ID NO: 24, (iii) a heavy chain variable domain including an amino acid sequence represented by SEQ ID NO: 27 and a light chain variable domain including an amino acid sequence represented by SEQ ID NO: 28, or (iv) a heavy chain domain including an amino acid sequence represented by SEQ ID NO: 347 and a light chain domain including an amino acid sequence represented by SEQ ID NO: 308.
[3] The method according to [1] or [2], in which p is 3 or 4.
[4] The method according to any one of [1] to [3], in which eribulin or the salt thereof is eribulin methanesulfonate.
[5] The method according to any one of [1] to [4], in which the step 1 is performed in the presence of a base.
[6] A method of producing a compound represented by Formula (B), 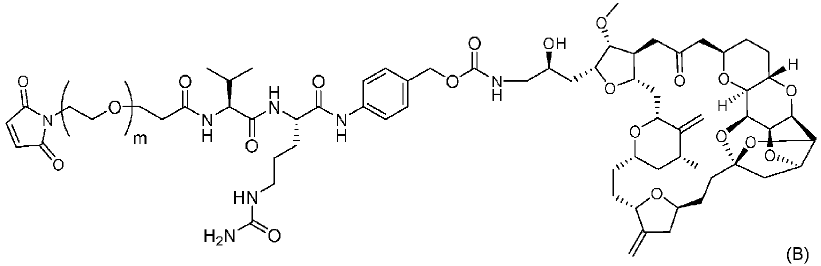
   in the formula, m is an integer of 1 to 10,
   the method including:
      a step of obtaining a compound represented by Formula (B) by reaction of eribulin or a salt thereof with a compound represented by Formula (A), 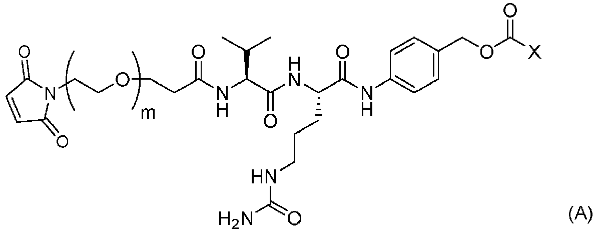
   in the formula, m is an integer of 1 to 10 and X is a phenoxy group or a nitrophenoxy group.
[7] The method according to [6], in which the step is performed in the presence of a base.
[8] A compound represented by Formula (A), 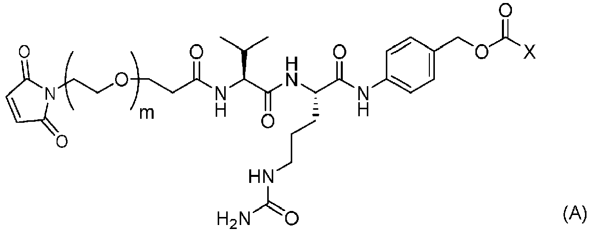 in the formula, m is an integer of 1 to 10 and X is a phenoxy group or a nitrophenoxy group.

### Advantageous Effects of Invention

The present invention can provide a method of producing an antibody-drug conjugate with high yield. In addition, the present invention can also provide a synthetic intermediate that is useful for producing an antibody-drug conjugate with high yield.

### Description of Embodiments

An embodiment of the present invention will be described below in detail.

The embodiment of the present invention relates to a method of producing an antibody-drug conjugate represented by Formula (I).

First, the ADC represented by Formula (I) will be described.

The ADC can bind, internalize, and kill tumor cells (for example, FRA-expressing tumor cells). In addition, it is preferable that an antibody moiety (Ab) used in the ADC is an antibody or an antigen-binding fragment thereof and targets tumor cells. The antibody or the antigen-binding fragment includes, for example:
(a) three heavy chain CDRs and three light chain CDRs defined by the Kabat numbering system (Kabat, Sequences of Proteins of Immunological Interest (National Institutes of Health, Bethesda, Md. (1987 and 1991))), the three heavy chain CDRs including amino acid sequences of a heavy chain complementarity determining region (heavy chain CDR) 1 represented by SEQ ID NO: 2, a heavy chain CDR2 represented by SEQ ID NO: 3, and a heavy chain CDR3 represented by SEQ ID NO: 4, and the three light chain CDRs including amino acid sequences of a light chain complementarity determining region (light chain CDR) 1 represented by SEQ ID NO: 7, a light chain CDR2 represented by SEQ ID NO: 8, and a light chain CDR3 represented by SEQ ID NO: 9; or
(b) three heavy chain CDRs and three light chain CDRs defined by the IMGT numbering system (International ImMunoGeneTics Information System (IMGT (registered trade name)), the three heavy chain CDRs including amino acid sequences of a heavy chain CDR1 represented by SEQ ID NO: 13, a heavy chain CDR2 represented by SEQ ID NO: 14, and a heavy chain CDR3 represented by SEQ ID NO: 15, and the three light chain CDRs including amino acid sequences of a light chain CDR1 represented by SEQ ID NO: 16, a light chain CDR2 represented by SEQ ID NO: 17, and a light chain CDR3 represented by SEQ ID NO: 18.

In the present specification, the terms "antibody-drug conjugate", "antibody conjugate", "conjugate", "immunoconjugate", and "ADC" are used interchangeably, and refer to a compound or derivative thereof that is linked to an antibody (for example, an anti-FRA antibody) and is defined by Formula I [in the formula, Ab is an antibody moiety (that is, an antibody or an antigen-binding fragment thereof), L is a linker moiety, D is eribulin, and p is the number of eribulin moieties per antibody moiety].

The term "antibody" is used in the broadest sense to refer to an immunoglobulin molecule that recognizes and specifically binds to a target, such as a protein, polypeptide, carbohydrate, polynucleotide, lipid, or combinations of the foregoing through at least one antigen recognition site within the variable domain of the immunoglobulin molecule. The heavy chain of an antibody is composed of a heavy chain variable domain (V_{H}) and a heavy chain constant domain (C_{H}). The light chain of an antibody is composed of a light chain variable domain (V_{L}) and a light chain constant domain (C_{L}). For the purposes of the present application, the mature heavy and light chain variable domains each include three complementarity determining regions (CDR1, CDR2 and CDR3) within four framework regions (FR1, FR2, FR3 and FR4) arranged from N-terminus to C-terminus: FR1, CDR1, FR2, CDR2, FR3, CDR3 and FR4. An "antibody" may be naturally occurring or man-made, such as monoclonal antibodies produced by conventional hybridoma technology. The term "antibody" includes full-length monoclonal antibodies, full-length polyclonal antibodies, and single chain antibodies. In addition, examples of an antigen-binding fragment of an antibody include Fab, Fab', F(ab')₂, and Fv. An antibody may be any one of the five major classes of immunoglobulins: IgA, IgD, IgE, IgG, and IgM, or subclasses thereof (for example, isotypes IgG1, IgG2, IgG3, IgG4). The term further encompasses human antibodies, chimeric antibodies, humanized antibodies, and any modified immunoglobulin molecule containing an antigen recognition site as long as they demonstrate the desired biological activities.

The term "monoclonal antibody", as used herein, refers to an antibody obtained from a population of substantially homogeneous antibodies, that is, the individual antibodies comprising the population are identical except for possible naturally occurring mutations that may be present in minor amounts. Monoclonal antibodies are highly specific, being directed against a single antigenic epitope. In contrast, conventional (polyclonal) antibody preparations typically include a multitude of antibodies directed against (or specific for) different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al. (1975) Nature 256:495, or may be made by recombinant DNA methods (for example, see U.S. Patent No. 4,816,567). Monoclonal antibodies may also be isolated from phage antibody libraries using the techniques described in Clackson et al. (1991) Nature 352:624-8, and Marks et al. (1991) J. Mol. Biol. 222:581-97, for example.

The monoclonal antibodies described herein specifically include "chimeric" antibodies, in which a portion of the heavy chain and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they specifically bind the target antigen and/or exhibit the desired biological activity.

The term "homologue" refers to a molecule which exhibits homology to another molecule, by for example, having sequences of chemical residues that are identical or similar at corresponding positions.

The term "human antibody", as used herein, refers an antibody produced by a human or an antibody having an amino acid sequence of an antibody produced by a human.

The term "chimeric antibody", as used herein, refers to antibodies where the amino acid sequence of the immunoglobulin molecule is derived from two or more species. In some instances, the variable domains of both heavy and light chains correspond to the variable domains of antibodies derived from one species with the desired specificity, affinity, and activity while the constant regions are homologous to antibodies derived from another species (for example, human) to minimize an immune response in the latter species.

As used herein, the term "humanized antibody" refers to forms of antibodies that contain sequences from non-human (for example, murine) antibodies as well as human antibodies. Such antibodies are chimeric antibodies which contain minimal sequence derived from non-human immunoglobulin. In general, the humanized antibody includes substantially all of at least one, and typically two, variable domains, in which all or substantially all of the hypervariable loops correspond to those of a non-human immunoglobulin and all or substantially all of the framework regions (FR) are those of a human immunoglobulin sequence. The humanized antibody optionally also includes at least a portion of an immunoglobulin constant region (Fc), typically a constant region (Fc) of a human immunoglobulin. The humanized antibody can be further modified by the substitution of residues, either in the Fv framework region and/or within the replaced non-human residues to refine and optimize antibody specificity, affinity, and/or activity.

The term "antigen-binding fragment" of an antibody, as used herein, refers to one or more fragments of an antibody that retain the ability to specifically bind to an antigen (for example, FRA). Antigen-binding fragments preferably also retain the ability to internalize into an antigen-expressing cell. In some embodiments, antigen-binding fragments also retain immune effector activity. It has been shown that fragments of a full-length antibody can perform the antigen-binding function of a full-length antibody. Examples of binding fragments encompassed within the term "antigen-binding fragment" of an antibody include: (i) a Fab fragment, a monovalent fragment consisting of the V_{L}, V_{H}, C_{L}, and C_{H1} domains; (ii) a F(ab')₂ fragment, a bivalent fragment including two Fab fragments linked by a disulfide bridge in the hinge region; (iii) a Fd fragment consisting of the V_{H} and C_{H1} domains; (iv) a Fv fragment consisting of the V_{L} and V_{H} domains of a single arm of an antibody; (v) a dAb fragment, which includes a single variable domain, for example, a V_{H} domain (for example, see Ward et al. (1989) Nature 341:544-6; and Winter et al. or WO 90/05144); and (vi) an isolated complementarity determining region (CDR). Furthermore, although the two domains of the Fv fragment, V_{L} and V_{H}, are encoded for by separate genes, they can be joined, using recombinant methods, by a synthetic linker that enables them to be made as a single protein chain in which the V_{L} and V_{H} domains pair to form monovalent molecules (known as single chain Fv (scFv)). See, for example, Bird et al. (1988) Science 242:423-6; and Huston et al. (1988) Proc. Natl. Acad. Sci. USA 85:5879-83. Such single chain antibodies are also intended to be encompassed within the term "antigen-binding fragment" of an antibody, and are known in the art as an exemplary type of binding fragment that can internalize into cells after binding. See, for example, Zhu et al. (2010) 9:2131-41; He et al. (2010) J. Nucl. Med. 51:427-32; and Fitting et al. (2015) MAbs 7:390-402. In certain embodiments, scFv molecules may be incorporated into a fusion protein. Other forms of single chain antibodies, such as diabodies are also encompassed. Diabodies are bispecific diabodies in which V_{H} and V_{L} domains are expressed on a single polypeptide chain, but using a linker that is too short to allow for pairing between the two regions on the same chain, thereby forcing the domains to pair with complementary regions of another chain and creating two antigen binding sites (for example, see Holliger et al. (1993) Proc. Natl. Acad. Sci. USA 90:6444-8; and Poljak et al. (1994) Structure 2:1121-3). Antigen-binding fragments are obtained using conventional techniques known to those of skill in the art, and the antigen-binding fragments are screened for utility (for example, binding affinity, internalization) in the same manner as are intact antibodies. Antigen-binding fragments may be prepared by cleavage of the intact protein, for example, by protease or chemical cleavage.

In the present specification, an antibody or an antigen-binding fragment thereof is an internalizing antibody or an internalizing antigen-binding fragment thereof. "Internalizing" as used herein in reference to an antibody or antigen-binding fragment refers to an antibody or antigen-binding fragment that is capable of being taken through the cell's lipid bilayer membrane to an internal compartment (that is, "internalized") after binding to the cell, preferably into a degradative compartment, such as lysosome, in the cell. For example, an internalizing anti-FRA antibody is one that is capable of being taken into the cell after binding to FRA on the cell membrane.

The term "folate receptor alpha" or "FRA", as used herein, refers to any native form of human FRA. The term encompasses full-length FRA (for example, NCBI Reference Sequence: NP_000793; SEQ ID NO: 19), as well as any form of human FRA that results from cellular processing. The term also encompasses naturally occurring variants of FRA, including but not limited to splice variants, allelic variants, and isoforms. FRA can be isolated from a human, or may be produced recombinantly or by synthetic methods.

The term "anti-FRA antibody" or "antibody that specifically binds to FRA" refers to any form of antibody or antigen-binding fragment thereof that specifically binds to FRA, and encompasses monoclonal antibodies (including full length monoclonal antibodies), polyclonal antibodies, and biologically functional antibody fragments as long as they specifically bind to FRA. Preferably the anti-FRA antibody used in the ADCs disclosed herein is an antibody or an antigen-binding fragment thereof. MORAb-003 is an exemplary anti-human FRA antibody. As used herein, the terms "specific", and "specifically binds" refer to the selective binding of the antibody to the target antigen epitope. Antibodies can be tested for specificity of binding by comparing binding to appropriate antigen to binding to irrelevant antigen or antigen mixture under a given set of conditions. If the antibody binds to the appropriate antigen with at least 2, 5, 7, and preferably 10 times more affinity than to irrelevant antigen or antigen mixture, then it is considered to be specific. In one embodiment, a specific antibody is one that only binds to the FRA antigen, but does not bind (or exhibits minimal binding) to other antigens.

The term "human epidermal growth factor receptor 2", "her2", or "her2/neu", as used herein, refers to any native form of human her2. The term encompasses full-length her2 (for example, NCBI Reference Sequence: NP_004439.2; SEQ ID NO: 21), as well as any form of human her2 that results from cellular processing. The term also encompasses naturally occurring variants of her2, including but not limited to splice variants, allelic variants, and isoforms. Her2 can be isolated from human, or may be produced recombinantly or by synthetic methods.

The term "anti-her2 antibody" or "antibody that specifically binds to her2" refers to any form of antibody or antigen-binding fragment thereof that specifically binds to her2, and encompasses monoclonal antibodies (including full length monoclonal antibodies), polyclonal antibodies, and biologically functional antibody fragments as long as they specifically bind to her2. U.S. Pat. No. 5,821,337 (incorporated herein by reference) provides exemplary her2-binding sequences, including exemplary anti-her2 antibody sequences. Preferably the anti-her2 antibody used in the ADCs disclosed herein is an antibody or an antigen-binding fragment thereof. Trastuzumab is an exemplary anti-human her2 antibody.

The term "epitope" refers to the portion of an antigen capable of being recognized and specifically bound by an antibody. When the antigen is a polypeptide, epitopes can be formed from contiguous amino acids or noncontiguous amino acids juxtaposed by tertiary folding of the polypeptide. The epitope bound by an antibody may be identified using any epitope mapping technique known in the art. Examples of the epitope mapping techniques include X-ray crystallography for epitope identification by direct visualization of the antigen-antibody complex, monitoring the binding of the antibody to fragments or mutated variations of the antigen, and monitoring solvent accessibility of different parts of the antibody and the antigen. Exemplary strategies used to map epitopes include, but are not limited to, array-based oligo-peptide scanning, limited proteolysis, site-directed mutagenesis, high-throughput mutagenesis mapping, hydrogen-deuterium exchange, and mass spectrometry (for example, see Gershoni et al. (2007) 21:145-56; and Hager-Braun and Tomer (2005) Expert Rev. Proteomics 2:745-56).

Competitive binding and epitope binning can also be used to determine antibodies sharing identical or overlapping epitopes. Competitive binding can be evaluated using a cross-blocking assay, such as the assay described in "Antibodies, A Laboratory Manual", Cold Spring Harbor Laboratory, Harlow and Lane (1st edition 1988, 2nd edition 2014). In some embodiments, binding of a test antibody or an antigen-binding fragment thereof is evaluated to be competitive when the test antibody or the antigen-binding fragment thereof reduces binding of a reference antibody or an antigen-binding fragment thereof to a target antigen such as FRA or her2 (for example, a binding protein including CDRs and/or variable domains selected from those identified in Tables 2, 4, and 6), by at least about 50% in the cross-blocking assay (for example, 50%, 60%, 70%, 80%, 90%, 95%, 99%, 99.5%, or more, or any percentage in between), and/or vice versa. In some embodiments, competitive binding can be due to identical or similar (for example, partially overlapping) epitopes, or due to steric hindrance where antibodies or antigen-binding fragments thereof bind to nearby epitopes. See, for example, Tzartos, Methods in Molecular Biology (Morris, ed. (1998) vol. 66, pp. 55-66). In some embodiments, competitive binding can be used to sort groups of binding proteins that share similar epitopes, for example, those that compete for binding can be "binned" as a group of binding proteins that have overlapping or nearby epitopes, while those that do not compete can be classified into a separate group of binding proteins that do not have overlapping or nearby epitopes.

The term "kₒₙ" or "kₐ" refers to the on rate constant for association of an antibody to the antigen to form the antibody/antigen complex. The rate can be determined using standard assays, such as a Biacore or ELISA assay.

The term "k_{off}" or "k_{d}" refers to the off rate constant for dissociation of an antibody from the antibody/antigen complex. The rate can be determined using standard assays, such as a Biacore or ELISA assay.

The term "K_{D}" refers to the equilibrium dissociation constant of a particular antibody-antigen interaction. K_{D} is calculated by kₐ/k_{d}. The rate can be determined using standard assays, such as a Biacore or ELISA assay.

The term "p" or "antibody:drug ratio" refers to the number of structural units including linker moieties and eribulin per antibody moiety (Ab) (that is, drug loading). In some embodiments, p is an integer of 1 to 10, 1 to 9, 1 to 8, 1 to 7, 1 to 6, 1 to 5, 1 to 4, 1 to 3, or 1 or 2 and preferably an integer of 3 or 4. In compositions including multiple copies of ADCs represented by Formula I, "p" refers to the average number of structural units including linker moieties and eribulin per antibody moiety (Ab) (also referred to as average drug loading). When p is represented by the average drug loading, p may be 3 to 4, 3.2 to 3.8, 3.5 to 4.5, 3.6 to 4.4, or 4.

In some embodiments, p is an integer of 1 to 6, 2 to 5, or 3 or 4. When p is a larger number, the number of eribulin moieties per antibody moiety increases. Therefore, a larger number of eribulin can be delivered to target cells with the single antibody, and the pharmacological effect thereof can be further increased.

When the ADC is present outside a cell, the ADC remains intact. When the ADC is internalized into a cell (for example, a cancer cell), the linker moiety of the ADC is cleaved, and eribulin in the cell is released. The linker moiety is typically stable outside a cell. That is, the ADC recognizes a cell (for example, a cancer cell) that expresses an antigen specific for the antibody moiety (Ab) and enters the cell. Regarding the ADC in the cell, the linker moiety that links eribulin and the antibody moiety (Ab) is cleaved such that eribulin is released and the pharmacological effect is exhibited.

### 1. Antibody Moiety (Ab)

The antibody moiety (Ab) in the ADC is an antibody or an antigen-binding fragment thereof, in particular, an anti-folate receptor alpha (FRA) antibody or an antigen-binding fragment thereof, and can bind to a FRA-expressing tumor cell.

In some embodiments, the antibody or the antigen-binding fragment thereof binds to a folate receptor alpha (FRA) and can target an FRA-expressing tumor cell. In addition, in some embodiments, the antibody or the antigen-binding fragment thereof includes: (a) three heavy chain CDRs (a heavy chain CDR1 represented by SEQ ID NO: 2, a heavy chain CDR2 represented by SEQ ID NO: 3, and a heavy chain CDR3 represented by SEQ ID NO: 4) and three light chain CDRs (a light chain CDR1 represented by SEQ ID NO: 7, a light chain CDR2 represented by SEQ ID NO: 8, and a light chain CDR3 represented by SEQ ID NO: 9) defined by the Kabat numbering system; or (b) three heavy chain CDRs (a heavy chain CDR1 represented by SEQ ID NO: 13, a heavy chain CDR2 represented by SEQ ID NO: 14, and a heavy chain CDR3 represented by SEQ ID NO: 15) and three light chain CDRs (a light chain CDR1 represented by SEQ ID NO: 16, a light chain CDR2 represented by SEQ ID NO: 17, and a light chain CDR3 represented by SEQ ID NO: 18) defined by the IMGT numbering system. In addition, in some embodiments, the antibody or the antigen-binding fragment thereof includes human framework sequences. In some embodiments, the antibody or the antigen-binding fragment thereof includes a heavy chain variable domain represented by SEQ ID NO: 23 and a light chain variable domain represented by SEQ ID NO: 24. In some embodiments, the antibody or the antigen-binding fragment includes a human IgG1 heavy chain constant domain and an Ig kappa light chain constant domain. In some embodiments, the antibody or the antigen-binding fragment competes for binding and/or binds to the same epitope as an antibody including a heavy chain variable domain represented by SEQ ID NO: 23 and a light chain variable domain represented by SEQ ID NO: 24. In some embodiments, the antibody or the antigen-binding fragment thereof binds to an epitope including alanine-histidine-lysine-aspartic acid (Ala-His-Lys-Asp, SEQ ID NO: 345) (Non-Patent Literature 9). In some embodiments, the antibody or the antigen-binding fragment binds to an epitope including NTSQEAHKDVSYL (asn-Thr-Ser-Gln-Glu-Ala-His-Lys-Asp-Val-Ser-Tyr-Leu, SEQ ID NO: 346).

In other embodiments, the antibody or the antigen-binding fragment thereof binds to human epidermal growth factor receptor 2 (her2) and can target her2-expressing tumor cells. In some embodiments, the antibody or the antigen-binding fragment thereof includes: (a) three heavy chain CDRs (a heavy chain CDR1 represented by SEQ ID NO: 71, a heavy chain CDR2 represented by SEQ ID NO: 72, and a heavy chain CDR3 represented by SEQ ID NO: 73) and three light chain CDRs (a light chain CDR1 represented by SEQ ID NO: 74, a light chain CDR2 represented by SEQ ID NO: 75, and a light chain CDR3 represented by SEQ ID NO: 76) defined by the Kabat numbering system; or (b) three heavy chain CDRs (a heavy chain CDR1 represented by SEQ ID NO: 191, a heavy chain CDR2 represented by SEQ ID NO: 192, and a heavy chain CDR3 represented by SEQ ID NO: 193) and three light chain CDRs (a light chain CDR1 represented by SEQ ID NO: 194, a light chain CDR2 represented by SEQ ID NO: 195, and a light chain CDR3 represented by SEQ ID NO: 196) defined by the IMGT numbering system. In addition, in some embodiments, the antibody or the antigen-binding fragment thereof includes human framework sequences. In some embodiments, the antibody or the antigen-binding fragment thereof includes a heavy chain variable domain represented by SEQ ID NO: 27 and a light chain variable domain represented by SEQ ID NO: 28. In some embodiments, the antibody or the antigen-binding fragment includes a human IgG1 heavy chain constant domain and an Ig kappa light chain constant domain. In some embodiments, the antibody or the antigen-binding fragment competes for binding and/or binds to the same epitope as an antibody including a heavy chain variable domain represented by SEQ ID NO: 27 and a light chain variable domain represented by SEQ ID NO: 28.

In other embodiments, the antibody or the antigen-binding fragment thereof binds to mesothelin (MSLN) and can target MSLN-expressing tumor cells. In some embodiments, the antibody or the antigen-binding fragment thereof includes: (a) three heavy chain CDRs (a heavy chain CDR1 represented by SEQ ID NO: 65, a heavy chain CDR2 represented by SEQ ID NO: 66, and a heavy chain CDR3 represented by SEQ ID NO: 67) and three light chain CDRs (a light chain CDR1 represented by SEQ ID NO: 68, a light chain CDR2 represented by SEQ ID NO: 69, and a light chain CDR3 represented by SEQ ID NO: 70) defined by the Kabat numbering system; or (b) three heavy chain CDRs (a heavy chain CDR1 represented by SEQ ID NO: 185, a heavy chain CDR2 represented by SEQ ID NO: 186, and a heavy chain CDR3 represented by SEQ ID NO: 187) and three light chain CDRs (a light chain CDR1 represented by SEQ ID NO: 188, a light chain CDR2 represented by SEQ ID NO: 189, and a light chain CDR3 represented by SEQ ID NO: 190) defined by the IMGT numbering system. In some embodiments, the antibody or the antigen-binding fragment includes a heavy chain variable domain represented by SEQ ID NO: 25 and a light chain variable domain represented by SEQ ID NO: 26. In some embodiments, the antibody or the antigen-binding fragment includes a human IgG1 heavy chain constant domain and an Ig kappa light chain constant domain. In some embodiments, the antibody or the antigen-binding fragment competes for binding and/or binds to the same epitope as an antibody including a heavy chain variable domain represented by SEQ ID NO: 25 and a light chain variable domain represented by SEQ ID NO: 26.

In the present embodiment, a preferable antibody moiety (Ab) is an anti-folate receptor alpha antibody or an antigen-binding fragment thereof including: (a) three heavy chain CDRs including amino acid sequences represented by SEQ ID NO: 2 (heavy chain CDR1), SEQ ID NO: 3 (heavy chain CDR2), and SEQ ID NO: 4 (heavy chain CDR3) or three light chain CDRs including amino acid sequences represented by SEQ ID NO: 7 (light chain CDR1), SEQ ID NO: 8 (light chain CDR2), and SEQ ID NO: 9 (light chain CDR3) defined by the Kabat numbering system; or (b) three heavy chain CDRs including amino acid sequences represented by SEQ ID NO: 13 (heavy chain CDR1), SEQ ID NO: 14 (heavy chain CDR2), and SEQ ID NO: 15 (heavy chain CDR3) or three light chain CDRs including amino acid sequences represented by SEQ ID NO: 16 (light chain CDR1), SEQ ID NO: 17 (light chain CDR2), and SEQ ID NO: 18 (light chain CDR3) defined by the IMGT numbering system. Another preferable antibody moiety (Ab) is an anti-human epidermal growth factor receptor 2 (her2) antibody or an antigen-binding fragment thereof including: (c) three heavy chain CDRs including amino acid sequences represented by SEQ ID NO: 71 (heavy chain CDR1), SEQ ID NO: 72 (heavy chain CDR2), and SEQ ID NO: 73 (heavy chain CDR3) or three light chain CDRs including amino acid sequences represented by SEQ ID NO: 74 (light chain CDR1), SEQ ID NO: 75 (light chain CDR2), and SEQ ID NO: 76 (light chain CDR3) defined by the Kabat numbering system; or (d) three heavy chain CDRs including amino acid sequences represented by SEQ ID NO: 191 (heavy chain CDR1), SEQ ID NO: 192 (heavy chain CDR2), and SEQ ID NO: 193 (heavy chain CDR3) or three light chain CDRs including amino acid sequences represented by SEQ ID NO: 194 (light chain CDR1), SEQ ID NO: 195 (light chain CDR2), and SEQ ID NO: 196 (light chain CDR3) defined by the IMGT numbering system. It is more preferable that the antibody moiety (Ab) is an anti-folate receptor alpha antibody or an antigen-binding fragment thereof.

The antibody moiety (Ab) includes any antibody or antigen-binding fragment that specifically binds to a target antigen on a cancer cell in the range. The antibody or the antigen-binding fragment can bind to a target antigen with a dissociation constant (K_{D}) of ≤ 1 mM, < 100 nM, or ≤ 10 nM, or any amount in between when measured by, for example, BIAcore (registered trade name) analysis. In certain embodiments, K_{D} is 1 pM to 500 pM. In some embodiments, K_{D} is 500 pM to 1 µM, 1 µM to 100 nm, or 100 mM to 10 nM.

In some embodiments, the antibody moiety is a four-chain antibody (also referred to as immunoglobulin) including two heavy chains and two light chains. In some embodiments, the antibody moiety is a two-chain half body (one light chain and one heavy chain) of immunoglobulin or an antigen-binding fragment thereof.

Amino acid and nucleic acid sequences of exemplary antibodies according to the present disclosure will be clearly shown in Tables 1 to 9.

**[Table 1]**

| mAb | Class/Isotype | Target |
|---|---|---|
| MORAb-003 | humanized | human folate receptor alpha |
| MORAb-009 | mouse-human chimeric | human mesothelin |
| trastuzumab | humanized | human her2/neu |
| 33011-xi | rabbit-human chimeric | human mesothelin |
| 33011-zu | humanized | human mesothelin |
| 111B10-xi | rabbit-human chimeric | human mesothelin |
| 111B10-zu | humanized | human mesothelin |
| 201C15-xi | rabbit-human chimeric | human mesothelin |
| 201C15-zu | humanized | human mesothelin |
| 346C6-xi | rabbit-human chimeric | human mesothelin |
| 346C6-zu | humanized | human mesothelin |

| | | |
|---|---|---|
| Abbreviations: xi is a chimeric antibody and zu is a humanized antibody. | | |

Amino acid sequences of mAb variable domains

**[Table 2]**

| | SEQ ID NO | mAb | IgGchain |
|---|---|---|---|
| 1 | 23 | MORAb-003 | Heavy chain |
| 2 | 24 | MORAb-003 | Light chain |
| 3 | 25 | MORAb-009 | Heavy chain |
| 4 | 26 | MORAb-009 | Light chain |
| 5 | 27 | trastuzumab | Heavy chain |
| 6 | 28 | trastuzumab | Light chain |
| 7 | 29 | 33011-xi | Heavy chain |
| 8 | 30 | 33011-xi | Light chain |
| 9 | 31 | 33011-zu | Heavy chain |
| 10 | 32 | 33011-zu | Light chain |
| 11 | 33 | 111B10-xi | Heavy chain |
| 12 | 34 | 111B10-xi | Light chain |
| 13 | 35 | 111B10-zu | Heavy chain |
| 14 | 36 | 111B10-zu | Light chain |
| 15 | 37 | 201C15-xi | Heavy chain |
| 16 | 38 | 201C15-xi | Light chain |
| 17 | 39 | 201C15-zu | Heavy chain |
| 18 | 40 | 201C15-zu | Light chain |
| 19 | 41 | 346C6-xi | Heavy chain |
| 20 | 42 | 346C6-xi | Light chain |
| 21 | 43 | 346C6-zu | Heavy chain |
| 22 | 44 | 346C6-zu | Light chain |

Nucleic acid sequences encoding mAb variable domains

**[Table 3]**

| | SEQ ID NO | mAb | IgG chain |
|---|---|---|---|
| 1 | 45 | MORAb-003 | Heavy chain |
| 2 | 46 | MORAb-003 | Light chain |
| 3 | 47 | MORAb-009 | Heavy chain |
| 4 | 48 | MORAb-009 | Light chain |
| 5 | 49 | 33011-xi | Heavy chain |
| 6 | 50 | 33011-xi | Light chain |
| 7 | 51 | 33011-zu | Heavy chain |
| 8 | 52 | 33011-zu | Light chain |
| 9 | 53 | 111B10-xi | Heavy chain |
| 10 | 54 | 111B10-xi | Light chain |
| 11 | 55 | 111B10-zu | Heavy chain |
| 12 | 56 | 111B10-zu | Light chain |
| 13 | 57 | 201C15-xi | Heavy chain |
| 14 | 58 | 201C15-xi | Light chain |
| 15 | 59 | 201C15-zu | Heavy chain |
| 16 | 60 | 201C15-zu | Light chain |
| 17 | 61 | 346C6-xi | Heavy chain |
| 18 | 62 | 346C6-xi | Light chain |
| 19 | 63 | 346C6-zu | Heavy chain |
| 20 | 64 | 346C6-zu | Light chain |

Amino acid sequences of mAb Kabat CDRs

**[Table 4]**

| | SEQ ID NO | mAb | IgG chain |
|---|---|---|---|
| 1 | 2 | MORAb-003 | HC CDR1 |
| 2 | 3 | MORAb-003 | HCCDR2 |
| 3 | 4 | MORAb-003 | HC CDR3 |
| 4 | 7 | MORAb-003 | LC CDR1 |
| 5 | 8 | MORAb-003 | LC CDR2 |
| 6 | 9 | MORAb-003 | LC CDR3 |
| 7 | 65 | MORAb-009 | HC CDR1 |
| 8 | 66 | MORAb-009 | HC CDR2 |
| 9 | 67 | MORAb-009 | HC CDR3 |
| 10 | 68 | MORAb-009 | LC CDR1 |
| 11 | 69 | MORAb-009 | LC CDR2 |
| 12 | 70 | MORAb-009 | LC CDR3 |
| 13 | 71 | trastuzumab | HC CDR1 |
| 14 | 72 | trastuzumab | HC CDR2 |
| 15 | 73 | trastuzumab | HC CDR3 |
| 16 | 74 | trastuzumab | LC CDR1 |
| 17 | 75 | trastuzumab | LC CDR2 |
| 18 | 76 | trastuzumab | LC CDR3 |
| 19 | 77 | 33011-xi | HC CDR1 |
| 20 | 78 | 33011-xi | HC CDR2 |
| 21 | 79 | 33011-xi | HC CDR3 |
| 22 | 80 | 33011-xi | LC CDR1 |
| 23 | 81 | 33011-xi | LC CDR2 |
| 24 | 82 | 33011-xi | LC CDR3 |
| 25 | 83 | 33011-zu | HC CDR1 |
| 26 | 84 | 33011-zu | HC CDR2 |
| 27 | 85 | 33011-zu | HC CDR3 |
| 28 | 86 | 33011-zu | LC CDR1 |
| 29 | 87 | 33011-zu | LC CDR2 |
| 30 | 88 | 33011-zu | LC CDR3 |
| 31 | 89 | 111B10-xi | HC CDR1 |
| 32 | 90 | 111B10-xi | HC CDR2 |
| 33 | 91 | 111B10-xi | HC CDR3 |
| 34 | 92 | 111B10-xi | LC CDR1 |
| 35 | 93 | 111B10-xi | LC CDR2 |
| 36 | 94 | 111B10-xi | LC CDR3 |
| 37 | 95 | 111B10-zu | HC CDR1 |
| 38 | 96 | 111B10-zu | HC CDR2 |
| 39 | 97 | 111B10-zu | HCCDR3 |
| 40 | 98 | 111B10-zu | LC CDR1 |
| 41 | 99 | 111B10-zu | LC CDR2 |
| 42 | 100 | 111B10-zu | LC CDR3 |
| 43 | 101 | 201C15-xi | HC CDR1 |
| 44 | 102 | 201C15-xi | HC CDR2 |
| 45 | 103 | 201C15-xi | HC CDR3 |
| 46 | 104 | 201C15-xi | LC CDR1 |
| 47 | 105 | 201C15-xi | LC CDR2 |
| 48 | 106 | 201C15-xi | LC CDR3 |
| 49 | 107 | 201C15-zu | HC CDR1 |
| 50 | 108 | 201C15-zu | HC CDR2 |
| 51 | 109 | 201C15-zu | HC CDR3 |
| 52 | 110 | 201C15-zu | LC CDR1 |
| 53 | 111 | 201C15-zu | LC CDR2 |
| 54 | 112 | 201C15-zu | LC CDR3 |
| 55 | 113 | 346C6-xi | HC CDR1 |
| 56 | 114 | 346C6-xi | HC CDR2 |
| 57 | 115 | 346C6-xi | HC CDR3 |
| 58 | 116 | 346C6-xi | LC CDR1 |
| 59 | 117 | 346C6-xi | LC CDR2 |
| 60 | 118 | 346C6-xi | LC CDR3 |
| 61 | 119 | 346C6-zu | HC CDR1 |
| 62 | 120 | 346C6-zu | HC CDR2 |
| 63 | 121 | 346C6-zu | HC CDR3 |
| 64 | 122 | 346C6-zu | LC CDR1 |
| 65 | 123 | 346C6-zu | LC CDR2 |
| 66 | 124 | 346C6-zu | LC CDR3 |

Nucleic acid sequences encoding mAb Kabat CDRs

**[Table 5]**

| | SEQ ID NO | mAb | IgG chain |
|---|---|---|---|
| 1 | 125 | MORAb-003 | HC CDR1 |
| 2 | 126 | MORAb-003 | HC CDR2 |
| 3 | 127 | MORAb-003 | HC CDR3 |
| 4 | 128 | MORAb-003 | LC CDR1 |
| 5 | 129 | MORAb-003 | LC CDR2 |
| 6 | 130 | MORAb-003 | LC CDR3 |
| 7 | 131 | MORAb-009 | HC CDR1 |
| 8 | 132 | MORAb-009 | HC CDR2 |
| 9 | 133 | MORAb-009 | HC CDR3 |
| 10 | 134 | MORAb-009 | LC CDR1 |
| 11 | 135 | MORAb-009 | LC CDR2 |
| 12 | 136 | MORAb-009 | LC CDR3 |
| 13 | 137 | 33011-xi | HC CDR1 |
| 14 | 138 | 33011-xi | HC CDR2 |
| 15 | 139 | 33011-xi | HC CDR3 |
| 16 | 140 | 33011-xi | LC CDR1 |
| 17 | 141 | 33011-xi | LC CDR2 |
| 18 | 142 | 33011-xi | LC CDR3 |
| 19 | 143 | 33011-zu | HC CDR1 |
| 20 | 144 | 33011-zu | HC CDR2 |
| 21 | 145 | 33011-zu | HC CDR3 |
| 22 | 146 | 33011-zu | LC CDR1 |
| 23 | 147 | 33011-zu | LC CDR2 |
| 24 | 148 | 33011-zu | LC CDR3 |
| 25 | 149 | 111B10-xi | HC CDR1 |
| 26 | 150 | 111B10-xi | HC CDR2 |
| 27 | 151 | 111B10-xi | HC CDR3 |
| 28 | 152 | 111B10-xi | LC CDR1 |
| 29 | 153 | 111B10-xi | LC CDR2 |
| 30 | 154 | 111B10-xi | LC CDR3 |
| 31 | 155 | 111B10-zu | HC CDR1 |
| 32 | 156 | 111B10-zu | HC CDR2 |
| 33 | 157 | 111B10-zu | HC CDR3 |
| 34 | 158 | 111B10-zu | LC CDR1 |
| 35 | 159 | 111B10-zu | LC CDR2 |
| 36 | 160 | 111B10-zu | LC CDR3 |
| 37 | 161 | 201C15-xi | HC CDR1 |
| 38 | 162 | 201C15-xi | HC CDR2 |
| 39 | 163 | 201C15-xi | HC CDR3 |
| 40 | 164 | 201C15-xi | LC CDR1 |
| 41 | 165 | 201C15-xi | LC CDR2 |
| 42 | 166 | 201C15-xi | LC CDR3 |
| 43 | 167 | 201C15-zu | HC CDR1 |
| 44 | 168 | 201C15-zu | HC CDR2 |
| 45 | 169 | 201C15-zu | HC CDR3 |
| 46 | 170 | 201C15-zu | LC CDR1 |
| 47 | 171 | 201C15-zu | LC CDR2 |
| 48 | 172 | 201C15-zu | LC CDR3 |
| 49 | 173 | 346C6-xi | HC CDR1 |
| 50 | 174 | 346C6-xi | HC CDR2 |
| 51 | 175 | 346C6-xi | HC CDR3 |
| 52 | 176 | 346C6-xi | LC CDR1 |
| 53 | 177 | 346C6-xi | LC CDR2 |
| 54 | 178 | 346C6-xi | LC CDR3 |
| 55 | 179 | 346C6-zu | HC CDR1 |
| 56 | 180 | 346C6-zu | HC CDR2 |
| 57 | 181 | 346C6-zu | HC CDR3 |
| 58 | 182 | 346C6-zu | LC CDR1 |
| 59 | 183 | 346C6-zu | LC CDR2 |
| 60 | 184 | 346C6-zu | LC CDR3 |

Amino acid sequences of mAb IMGT CDRs

**[Table 6]**

| | SEQ ID NO | mAb | IgG chain |
|---|---|---|---|
| 1 | 13 | MORAb-003 | HC CDR1 |
| 2 | 14 | MORAb-003 | HC CDR2 |
| 3 | 15 | MORAb-003 | HC CDR3 |
| 4 | 16 | MORAb-003 | LC CDR1 |
| 5 | 17 | MORAb-003 | LC CDR2 |
| 6 | 18 | MORAb-003 | LC CDR3 |
| 7 | 185 | MORAb-009 | HC CDR1 |
| 8 | 186 | MORAb-009 | HC CDR2 |
| 9 | 187 | MORAb-009 | HC CDR3 |
| 10 | 188 | MORAb-009 | LC CDR1 |
| 11 | 189 | MORAb-009 | LC CDR2 |
| 12 | 190 | MORAb-009 | LC CDR3 |
| 13 | 191 | trastuzumab | HC CDR1 |
| 14 | 192 | trastuzumab | HC CDR2 |
| 15 | 193 | trastuzumab | HC CDR3 |
| 16 | 194 | trastuzumab | LC CDR1 |
| 17 | 195 | trastuzumab | LC CDR2 |
| 18 | 196 | trastuzumab | LC CDR3 |
| 19 | 197 | 33011-xi | HC CDR1 |
| 20 | 198 | 33011-xi | HC CDR2 |
| 21 | 199 | 33011-xi | HC CDR3 |
| 22 | 200 | 33011-xi | LC CDR1 |
| 23 | 201 | 33011-xi | LC CDR2 |
| 24 | 202 | 33011-xi | LC CDR3 |
| 25 | 203 | 33011-zu | HC CDR1 |
| 26 | 204 | 33011-zu | HC CDR2 |
| 27 | 205 | 33011-zu | HC CDR3 |
| 28 | 206 | 33011-zu | LC CDR1 |
| 29 | 207 | 33011-zu | LC CDR2 |
| 30 | 208 | 33011-zu | LC CDR3 |
| 31 | 209 | 111B10-xi | HC CDR1 |
| 32 | 210 | 111B10-xi | HC CDR2 |
| 33 | 211 | 111B10-xi | HC CDR3 |
| 34 | 212 | 111B10-xi | LC CDR1 |
| 35 | 213 | 111B10-xi | LC CDR2 |
| 36 | 214 | 111B10-xi | LC CDR3 |
| 37 | 215 | 111B10-zu | HC CDR1 |
| 38 | 216 | 111B10-zu | HC CDR2 |
| 39 | 217 | 111B10-zu | HC CDR3 |
| 40 | 218 | 111B10-zu | LC CDR1 |
| 41 | 219 | 111B10-zu | LC CDR2 |
| 42 | 220 | 111B10-zu | LC CDR3 |
| 43 | 221 | 201C15-xi | HC CDR1 |
| 44 | 222 | 201C15-xi | HC CDR2 |
| 45 | 223 | 201C15-xi | HC CDR3 |
| 46 | 224 | 201C15-xi | LC CDR1 |
| 47 | 225 | 201C15-xi | LC CDR2 |
| 48 | 226 | 201C15-xi | LC CDR3 |
| 49 | 227 | 201C15-zu | HC CDR1 |
| 50 | 228 | 201C15-zu | HC CDR2 |
| 5! | 229 | 201C15-zu | HC CDR3 |
| 52 | 230 | 201C15-zu | LC CDR1 |
| 53 | 231 | 201C15-zu | LC CDR2 |
| 54 | 232 | 201C15-zu | LC CDR3 |
| 55 | 233 | 346C6-xi | HC CDR1 |
| 56 | 234 | 346C6-xi | HC CDR2 |
| 57 | 235 | 346C6-xi | HC CDR3 |
| 58 | 236 | 346C6-xi | LC CDR1 |
| 59 | 237 | 346C6-xi | LC CDR2 |
| 60 | 238 | 346C6-xi | LC CDR3 |
| 6! | 239 | 346C6-zu | HC CDR1 |
| 62 | 240 | 346C6-zu | HC CDR2 |
| 63 | 241 | 346C6-zu | HC CDR3 |
| 64 | 242 | 346C6-zu | LC CDR1 |
| 65 | 243 | 346C6-zu | LC CDR2 |
| 66 | 244 | 346C6-zu | LC CDR3 |

Nucleic acid sequences encoding mAb IMGT CDRs

**[Table 7]**

| | SEQ ID NO | mAb | IgG chain |
|---|---|---|---|
| 1 | 245 | MORAb-003 | HC CDR1 |
| 2 | 246 | MORAb-003 | HC CDR2 |
| 3 | 247 | MORAb-003 | HC CDR3 |
| 4 | 248 | MORAb-003 | LC CDR1 |
| 5 | 249 | MORAb-003 | LC CDR2 |
| 6 | 250 | MORAb-003 | LC CDR3 |
| 7 | 251 | MORAb-009 | HC CDR1 |
| 8 | 252 | MORAb-009 | HC CDR2 |
| 9 | 253 | MORAb-009 | HC CDR3 |
| 10 | 254 | MORAb-009 | LC CDR1 |
| 11 | 255 | MORAb-009 | LC CDR2 |
| 12 | 256 | MORAb-009 | LC CDR3 |
| 13 | 257 | 33011-xi | HC CDR1 |
| 14 | 258 | 33011-xi | HC CDR2 |
| 15 | 259 | 33011-xi | HC CDR3 |
| 16 | 260 | 33011-xi | LC CDR1 |
| 17 | 261 | 33011-xi | LC CDR2 |
| 18 | 262 | 33011-xi | LC CDR3 |
| 19 | 263 | 33011-zu | HC CDR1 |
| 20 | 264 | 33011-zu | HC CDR2 |
| 21 | 265 | 33011-zu | HC CDR3 |
| 22 | 266 | 33011-zu | LC CDR1 |
| 23 | 267 | 33011-zu | LC CDR2 |
| 24 | 268 | 33011-zu | LC CDR3 |
| 25 | 269 | 111B10-xi | HC CDR1 |
| 26 | 270 | 111B10-xi | HC CDR2 |
| 27 | 271 | 111B10-xi | HC CDR3 |
| 28 | 272 | 111B10-xi | LC CDR1 |
| 29 | 273 | 111B10-xi | LC CDR2 |
| 30 | 274 | 111B10-xi | LC CDR3 |
| 31 | 275 | 111B10-zu | HC CDR1 |
| 32 | 276 | 111B10-zu | HC CDR2 |
| 33 | 277 | 111B10-zu | HC CDR3 |
| 34 | 278 | 111B10-zu | LC CDR1 |
| 35 | 279 | 111B10-zu | LC CDR2 |
| 36 | 280 | 111B10-zu | LC CDR3 |
| 37 | 281 | 201C15-xi | HC CDR1 |
| 38 | 282 | 201C15-xi | HC CDR2 |
| 39 | 283 | 201C15-xi | HC CDR3 |
| 40 | 284 | 201C15-xi | LC CDR1 |
| 41 | 285 | 201C15-xi | LC CDR2 |
| 42 | 286 | 201C15-xi | LC CDR3 |
| 43 | 287 | 201C15-zu | HC CDR1 |
| 44 | 288 | 201C15-zu | HC CDR2 |
| 45 | 289 | 201C15-zu | HC CDR3 |
| 46 | 290 | 201C15-zu | LC CDR1 |
| 47 | 291 | 201C15-zu | LC CDR2 |
| 48 | 292 | 201C15-zu | LC CDR3 |
| 49 | 293 | 346C6-xi | HC CDR1 |
| 50 | 294 | 346C6-xi | HC CDR2 |
| 51 | 295 | 346C6-xi | HC CDR3 |
| 52 | 296 | 346C6-xi | LC CDR1 |
| 53 | 297 | 346C6-xi | LC CDR2 |
| 54 | 298 | 346C6-xi | LC CDR3 |
| 55 | 299 | 346C6-zu | HC CDR1 |
| 56 | 300 | 346C6-zu | HC CDR2 |
| 57 | 301 | 346C6-zu | HC CDR3 |
| 58 | 302 | 346C6-zu | LC CDR1 |
| 59 | 303 | 346C6-zu | LC CDR2 |
| 60 | 304 | 346C6-zu | LC CDR3 |

Amino acid sequences of full-length mAb Ig chains

**[Table 8]**

| | SEQ ID NO | mAb | IgG chain |
|---|---|---|---|
| 1 | 1 | MORAb-003 | Heavy chain |
| 2 | 6 | MORAb-003 | Light chain |
| 3 | 305 | MORAb-009 | Heavy chain |
| 4 | 306 | MORAb-009 | Light chain |
| 5 | 307 | trastuzumab | Heavy chain |
| 6 | 308 | trastuzumab | Light chain |
| 7 | 309 | 33011-xi | Heavy chain |
| 8 | 310 | 33011-xi | Light chain |
| 9 | 311 | 33011-zu | Heavy chain |
| 10 | 312 | 33011-zu | Light chain |
| 11 | 313 | 111B10-xi | Heavy chain |
| 12 | 314 | 111B10-xi | Light chain |
| 13 | 315 | 111B10-zu | Heavy chain |
| 14 | 316 | 111B10-zu | Light chain |
| 15 | 317 | 201C15-xi | Heavy chain |
| 16 | 318 | 201C15-xi | Light chain |
| 17 | 319 | 201C15-zu | Heavy chain |
| 18 | 320 | 201C15-zu | Light chain |
| 19 | 321 | 346C6-xi | Heavy chain |
| 20 | 322 | 346C6-xi | Light chain |
| 21 | 323 | 346C6-zu | Heavy chain |
| 22 | 324 | 346C6-zu | Light chain |
| 23 | 347 | trastuzumab | Heavy chain |

Nucleic acid sequences encoding full-length mAb Ig chains

**[Table 9]**

| | SEQ ID NO | mAb | IgG chain |
|---|---|---|---|
| 1 | 325 | MORAb-003 | Heavy chain |
| 2 | 326 | MORAb-003 | Light chain |
| 3 | 327 | MORAb-009 | Heavy chain |
| 4 | 328 | MORAb-009 | Light chain |
| 5 | 329 | 33011-xi | Heavy chain |
| 6 | 330 | 33011-xi | Light chain |
| 7 | 331 | 33011-zu | Heavy chain |
| 8 | 332 | 33011-zu | Light chain |
| 9 | 333 | 111B10-xi | Heavy chain |
| 10 | 334 | 111B10-xi | Light chain |
| 11 | 335 | 111B10-zu | Heavy chain |
| 12 | 336 | 111B10-zu | Light chain |
| 13 | 337 | 201C15-xi | Heavy chain |
| 14 | 338 | 201C15-xi | Light chain |
| 15 | 339 | 201C15-zu | Heavy chain |
| 16 | 340 | 201C15-zu | Light chain |
| 17 | 341 | 346C6-xi | Heavy chain |
| 18 | 342 | 346C6-xi | Light chain |
| 19 | 343 | 346C6-zu | Heavy chain |
| 20 | 344 | 346C6-zu | Light chain |

The exemplary sequences do not include leader sequences.

The ADC may include any set of heavy and light chain variable domains listed in the tables above (for example, MORAb-003 heavy and light chain variable domains, or trastuzumab heavy and light chain variable domains), or a set of six CDR sequences from the heavy and light chain set. In some embodiments, the ADC includes human heavy and light chain constant regions or fragments thereof. For example, the ADC may include a human IgG heavy chain constant domain (for example, IgG1) and a human kappa or lambda light chain constant domain. The antibody moiety includes a human immunoglobulin G subtype 1 (IgG1) heavy chain constant region together a human Ig kappa light chain constant region.

A target cancer antigen of the ADC may be a folate receptor alpha (FRA).

The anti-FRA antibody or the antigen-binding fragment thereof may include three heavy chain CDRs (a heavy chain CDR1 represented by SEQ ID NO: 2, a heavy chain CDR2 represented by SEQ ID NO: 3, and a heavy chain CDR3 represented by SEQ ID NO: 4) and three light chain CDRs (a light chain CDR1 represented by SEQ ID NO: 7, a light chain CDR2 represented by SEQ ID NO: 8, and a light chain CDR3 represented by SEQ ID NO: 9) defined by the Kabat numbering system.

In addition, the anti-FRA antibody or the antigen-binding fragment thereof may include three heavy chain CDRs (a heavy chain CDR1 represented by SEQ ID NO: 13, a heavy chain CDR2 represented by SEQ ID NO: 14, and a heavy chain CDR3 represented by SEQ ID NO: 15) and three light chain CDRs (a light chain CDR1 represented by SEQ ID NO: 16, a light chain CDR2 represented by SEQ ID NO: 17, and a light chain CDR3 represented by SEQ ID NO: 18) defined by the IMGT numbering system.

In various embodiments, the anti-FRA antibody or the antigen-binding fragment thereof includes: a heavy chain variable domain including an amino acid sequence represented by SEQ ID NO: 23 and a light chain variable domain including an amino acid sequence represented by SEQ ID NO: 24; or a heavy chain variable domain including an amino acid sequence represented by SEQ ID NO: 1 and a light chain variable domain including an amino acid sequence represented by SEQ ID NO: 6. In some embodiments, the anti-FRA antibody or the antigen-binding fragment thereof includes a heavy chain variable domain amino acid sequence represented by SEQ ID NO: 23 and a light chain variable domain amino acid sequence represented by SEQ ID NO: 24, or includes sequences that are at least 95% identical to the above-described sequences. In some embodiments, the anti-FRA antibody or the antigen-binding fragment thereof includes a heavy chain variable domain amino acid sequence that is at least 96%, at least 97%, at least 98%, or at least 99% identical to SEQ ID NO: 23 and a light chain variable domain amino acid sequence that is at least 96%, at least 97%, at least 98%, or at least 99% identical to SEQ ID NO: 24.

The anti-FRA antibody may include a human IgG1 heavy chain constant domain together with a human Ig kappa light chain constant domain.

The anti-FRA antibody includes a heavy chain amino acid sequence represented by SEQ ID NO: 1 or a sequence that is at least 95% identical to SEQ ID NO: 1 and a light chain amino acid sequence represented by SEQ ID NO: 6 or a sequence that is at least 95% identical to SEQ ID NO: 6. In certain embodiments, the antibody includes a heavy chain amino acid sequence represented by SEQ ID NO: 1 and a light chain amino acid sequence represented by SEQ ID NO: 6, or includes sequences that are at least 95% identical to the above-described sequences. In some embodiments, the anti-FRA antibody includes a heavy chain amino acid sequence that is at least 96%, at least 97%, at least 98%, or at least 99% identical to SEQ ID NO: 1 and/or a light chain amino acid sequence that is at least 96%, at least 97%, at least 98%, or at least 99% identical to SEQ ID NO: 6. In some embodiments, the anti-FRA antibody includes a heavy chain that is encoded by a nucleotide sequence represented by SEQ ID NO: 11 (including nucleotides encoding a leader sequence) or SEQ ID NO: 325 (not including nucleotides encoding a leader sequence) and a light chain that is encoded by a nucleotide represented by SEQ ID NO: 12 (including nucleotides encoding a leader sequence) or SEQ ID NO: 326 (not including nucleotides encoding a leader sequence). In some embodiments, the heavy chain amino acid sequence lacks the C-terminal lysine. In various embodiments, the anti-FRA antibody has the amino acid sequence of the antibody produced by a cell line deposited under terms in accordance with the Budapest Treaty with the American Type Culture Collection (ATCC, 10801 University Blvd., Manassas, Va. 20110-2209) on April 24, 2006, under the Accession No. PTA-7552, or such sequences lacking the heavy chain C-terminal lysine. In various embodiments, the anti-FRA antibody is MORAb-003 (USAN name: farletuzumab) (Ebel et al. (2007) Cancer Immunity 7:6), or an antigen-binding fragment thereof.

A target cancer antigen of the ADC may be a human epidermal growth factor receptor 2 (her2).

In addition, the anti-her2 antibody or the antigen-binding fragment thereof may include three heavy chain CDRs (a heavy chain CDR1 represented by SEQ ID NO: 71, a heavy chain CDR2 represented by SEQ ID NO: 72, and a heavy chain CDR3 represented by SEQ ID NO: 73) and three light chain CDRs (a light chain CDR1 represented by SEQ ID NO: 74, a light chain CDR2 represented by SEQ ID NO: 75, and a light chain CDR3 represented by SEQ ID NO: 76) defined by the Kabat numbering system.

In addition, the anti-her2 antibody or the antigen-binding fragment thereof may include three heavy chain CDRs (a heavy chain CDR1 represented by SEQ ID NO: 191, a heavy chain CDR2 represented by SEQ ID NO: 192, and a heavy chain CDR3 represented by SEQ ID NO: 193) and three light chain CDRs (a light chain CDR1 represented by SEQ ID NO: 194, a light chain CDR2 represented by SEQ ID NO: 195, and a light chain CDR3 represented by SEQ ID NO: 196) defined by the IMGT numbering system.

In various embodiments, the anti-her2 antibody or the antigen-binding fragment thereof includes: a heavy chain variable domain including an amino acid sequence represented by SEQ ID NO: 27 and a light chain variable domain including an amino acid sequence represented by SEQ ID NO: 28; or a heavy chain domain including an amino acid sequence represented by SEQ ID NO: 347 and a light chain domain including an amino acid sequence represented by SEQ ID NO: 308. In some embodiments, the anti-her2 antibody or the antigen-binding fragment thereof includes a heavy chain variable domain amino acid sequence represented by SEQ ID NO: 27 and a light chain variable domain amino acid sequence represented by SEQ ID NO: 28, or include sequences that are at least 95% identical to the above-described sequences. In some embodiments, the anti-her2 antibody or the antigen-binding fragment thereof includes a heavy chain variable domain amino acid sequence that is at least 96%, at least 97%, at least 98%, or at least 99% identical to SEQ ID NO: 27 and/or a light chain variable domain amino acid sequence that is at least 96%, at least 97%, at least 98%, or at least 99% identical to SEQ ID NO: 28.

The anti-her2 antibody may include a human IgG1 heavy chain constant domain and a human Ig kappa light chain constant domain.

In various embodiments, the anti-her2 antibody includes a heavy chain amino acid sequence represented by SEQ ID NO: 307 or a sequence that is at least 95% identical to SEQ ID NO: 307 and a light chain amino acid sequence represented by SEQ ID NO: 308 or a sequence that is at least 95% identical to SEQ ID NO: 308. In certain embodiments, the antibody includes a heavy chain amino acid sequence represented by SEQ ID NO: 307 and a light chain amino acid sequence represented by SEQ ID NO: 308, or includes sequences that are at least 95% identical to the above-described sequences. In some embodiments, the anti-her2 antibody includes a heavy chain amino acid sequence that is at least 96%, at least 97%, at least 98%, or at least 99% identical to SEQ ID NO: 307 and a light chain amino acid sequence that is at least 96%, at least 97%, at least 98%, or at least 99% identical to SEQ ID NO: 308. In various embodiments, the anti-her2 antibody is trastuzumab or an antigen-binding fragment thereof.

In various embodiments, the anti-FRA antibody or the antigen-binding fragment thereof includes the three heavy chain CDRs and three light chain CDRs of MORAb-003 or includes amino acid sequences obtained by performing addition, deletion, or substitution of one or less, two or less, three or less, four or less, five or less, or six or less amino acids on a heavy chain CDR1 (SEQ ID NO: 2 defined by Kabat system or SEQ ID NO: 13 defined by IMGT system), a heavy chain CDR2 (SEQ ID NO: 3 defined by Kabat system or SEQ ID NO: 14 defined by IMGT system), a heavy chain CDR3 (SEQ ID NO: 4 defined by Kabat system or SEQ ID NO: 15 defined by IMGT system), a light chain CDR1 (SEQ ID NO: 7 defined by Kabat system or SEQ ID NO: 16 defined by IMGT system), a light chain CDR2 (SEQ ID NO: 8 defined by Kabat system or SEQ ID NO: 17 defined by IMGT system), and a light chain CDR3 (SEQ ID NO: 9 defined by Kabat system or SEQ ID NO: 18 defined by IMGT system).

In various other embodiments, the anti-her2 antibody or the antigen-binding fragment thereof includes the three heavy chain CDRs and three light chain CDRs of trastuzumab or includes amino acid sequences obtained by performing addition, deletion, or substitution of one or less, two or less, three or less, four or less, five or less, or six or less amino acids on a heavy chain CDR1 (SEQ ID NO: 71 defined by Kabat system or SEQ ID NO: 191 defined by IMGT system), a heavy chain CDR2 (SEQ ID NO: 72 defined by Kabat system or SEQ ID NO: 192 defined by IMGT system), a heavy chain CDR3 (SEQ ID NO: 73 defined by Kabat system or SEQ ID NO: 193 defined by IMGT system), a light chain CDR1 (SEQ ID NO: 74 defined by Kabat system or SEQ ID NO: 194 defined by IMGT system), a light chain CDR2 (SEQ ID NO: 75 defined by Kabat system or SEQ ID NO: 195 defined by IMGT system), and a light chain CDR3 (SEQ ID NO: 76 defined by Kabat system or SEQ ID NO: 196 defined by IMGT system).

In various embodiments, amino acid substitution is substitution of a single residue. Insertion is usually insertion of about 1 to about 20 amino acid residues, although considerably larger insertion may be tolerable as long as biological function is retained (for example, binding to FRA or her2). Deletion is usually deletion in a range from about 1 to about 20 amino acid residues, although in some cases deletions may be much larger. Substitution, deletion, insertion, or any combination thereof may be used to arrive at a final derivative or variant. Generally these changes are made on a few amino acids to minimize the alteration of the molecule, particularly the immunogenicity and specificity of the antigen binding protein. However, a larger change may be tolerable in certain circumstances. Conservative substitution is generally made in accordance with the following chart shown in Table 10.

**[Table 10]**

| Original Residue | Exemplary Substitution |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| He | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

A substantial change in function or immunological identity is made by selecting substitution that are less conservative than those shown in Table 10. For example, substitution may be made which more significantly affect: the structure of the polypeptide backbone in the area of the alteration, for example the α-helical or β-sheet structure; the charge or hydrophobicity of the molecule at the target site; or the bulk of the side chain. The substitutions which in general are expected to produce the greatest changes in the polypeptide's properties are the following (a) to (d):
(a) a hydrophilic amino acid residue (for example, Ser or Thr) is substituted by a hydrophobic amino acid residue (for example, Leu, Ile, Phe, Val, or Ala);
(b) Cys or Pro is substituted by any other residue;
(c) an amino acid residue having an electropositive side chain (for example, Lys, Arg, or His) is substituted by an electronegative amino acid residue (for example, Gln or Asn); and
(d) a residue having a bulky side chain (for example, Phe) is substituted by an amino acid not having a side chain (for example, Gly).

In various embodiments where variant antibody sequences are used in an ADC, the variants typically exhibit the same qualitative biological activity and elicit the same immune response, although variants may also be selected to modify the characteristics of the antigen binding proteins as needed. Alternatively, the variant may be designed such that the biological activity of the antigen binding protein is altered. For example, glycosylation sites may be altered or removed, as discussed herein.

In the ADC according to the present embodiment, various antibodies may be used to target cancer cells. Suitable antigens expressed on tumor cells but not healthy cells, or expressed on tumor cells at a higher level than on healthy cells, are known in the art, as are antibodies directed against them. These antibodies can be used together with the linker and eribulin disclosed herein.

The antibody moiety in the ADC may be an FRA-targeting antibody moiety such as MORAb-003. In some embodiments, the linker and eribulin according to the present disclosure may be surprisingly effective in several different tumor-targeting antibodies. When the antibody moiety in the ADC is an FRA-targeting antibody moiety such as MORAb-003, the ADC may bring about particularly improvement of drug: antibody ratio, tumor targeting, bystander killing, and treatment efficacy, and reduction in off-target killing. The improvement of treatment efficacy can be measured in vitro or in vivo, and may include reduction in tumor growth rate and/or reduction in tumor volume.

When the antibody moiety in the ADC is a her2-targeting antibody moiety such as trastuzumab, some or all of the favorable functional properties thereof are observed. In addition, the antibody moiety in the ADC may be a her2-targeting antibody moiety such as trastuzumab. When the antibody moiety in the ADC is a MSLN-targeting antibody moiety such as MORAb-009, some or all of the favorable functional properties thereof are observed.

In some embodiments, free cysteine residues are introduced into the amino acid sequence of the antibody moiety. For example, antibodies (cysteine-modified antibodies) in which one or more amino acids in an amino acid sequence of a parent antibody are replaced with cysteine can be prepared. For example, by introducing cysteine into a fragment of a parent FAb antibody, a cysteine-modified Fab antibody (also referred to as "ThioFab") can be formed. Similarly, by introducing cysteine into a parent monoclonal antibody, a cysteine-modified monoclonal antibody (also referred to as "ThioMab") can be formed. A single site mutation yields a single modified cysteine residue in a ThioFab, whereas a single site mutation yields two modified cysteine residues in a ThioMab, due to the dimeric nature of the IgG antibody. DNA encoding an amino acid sequence variant of the parent polypeptide can be prepared by a variety of methods known in the art (see, for example, the methods described in WO2006/034488). These methods include, but are not limited to, preparation by site-directed (or oligonucleotide-mediated) mutagenesis, PCR mutagenesis, and cassette mutagenesis of an earlier prepared DNA encoding the polypeptide. Variants of recombinant antibodies may also be constructed also by restriction fragment manipulation or by overlap extension PCR with synthetic oligonucleotides. The ADC represented by Formula I includes but are not limited to, antibodies that have one, two, three, or four modified cysteines (Lyon et al. (2012) Methods Enzymol. 502:123-38). In some embodiments, when one or more free cysteine residues are already present in the antibody moiety, the existing free cysteine residues may be used to conjugate the antibody moiety to eribulin without being modified.

### 2. Linker Moiety

The linker moiety in the present embodiment has the following chemical structure and is composed of five units including a maleimide unit (Mal), an oxyethylene unit (PEG), a propionic acid unit (PA), an amino acid unit (Val-Cit), and a self-immolative unit (pAB: p-aminobenzyloxycarbonyl) from the side adjacent to the antibody moiety.

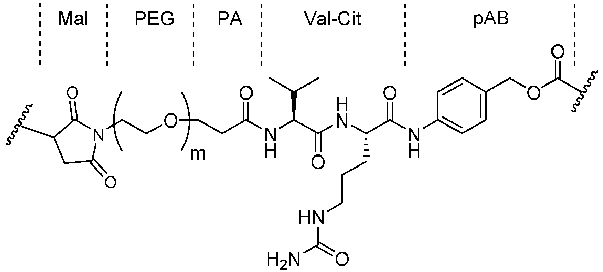

The linker moiety is stable outside a cell so as to be therapeutically sufficiently effective. In some embodiments, the linker moiety is stable outside a cell, such that the ADC remains intact when present in extracellular conditions (for example, prior to transport or delivery into a cell). The term "intact", used in the context of an ADC, means that the antibody moiety remains bound to eribulin. As used herein, the term "stable", in the context of an ADC, means that 20% or lower, about 15% or lower, about 10% or lower, about 5% or lower, about 3% or lower, or about 1% or lower of the linker (or any percentage in between) in a sample of ADC is cleaved (or in the case of an overall ADC is otherwise not intact) when the ADC is present in extracellular conditions.

Whether the linker moiety is stable extracellularly can be determined, for example, by including an ADC in plasma for a predetermined time period (for example, 2, 4, 6, 8, 16, or 24 hours) and then quantifying the amount of free drug moiety present in the plasma. Stability may allow the ADC time to localize to target tumor cells and prevent the premature release of the drug, which could lower the therapeutic index of the ADC by indiscriminately damaging both normal and tumor tissues. The linker moiety is stable outside of a target cell and releases eribulin from the ADC inside of the cell, such that eribulin can bind to its target (for example, to microtubules). Thus, the linker moiety according to the present embodiment: (i) maintains the specific binding properties of the antibody moiety; (ii) allows delivery, for example, intracellular delivery, of eribulin via stable binding to the antibody moiety; (iii) remains stable and intact until the ADC has been transported or delivered to its target site; and (iv) allows for the therapeutic effect, for example, cytotoxic effect, of eribulin after cleavage.

The linker is cleavable under intracellular conditions, such that cleavage of the linker sufficiently releases eribulin from the antibody moiety in the intracellular environment to activate eribulin and/or render the drug therapeutically effective. In some embodiments, eribulin is not cleaved from the antibody moiety until the ADC enters a cell that expresses an antigen specific for the antibody moiety of the ADC, and eribulin is cleaved from the antibody moiety after entering the cell. In some embodiments, the linker includes a cleavable moiety that is positioned such that no part of the linker or the antibody moiety remains bound to eribulin after cleavage.

The linker moiety includes an amino acid unit of valine-citrulline (Val-Cit). The amino acid unit of valine-citrulline (Val-Cit) is cleavable by a cleaving agent (for example, an intracellular peptidase or protease enzyme) that is present in the intracellular environment (for example, within a lysosome or endosome or caveolea). Peptidase such as cathepsin (for example, cathepsin B, C, F, H, K, L, O, S, V, X, or W) can cleave a valine-citrulline (Val-Cit) sequence or an alanine-alanine-asparagine (Ala-Ala-Asn) sequence (Dubowchik et al. (2002) Bioconjugate Chem. 13: 855-69). The linker moiety is cleaved by an enzyme in a target cell such that the ADC is separated into the antibody side and the eribulin side. The ADC including Val-Cit allows increased stability, decreased off-target cell killing, increased on-target cell killing, lower aggregation levels, and/or higher drug loading relative to an ADC including an alternate amino acid unit (for example, Gly-Gly).

Citrulline and eribulin are linked via p-aminobenzyloxycarbonyl (pAB). Specifically, an amino group of p-aminobenzyloxycarbonyl forms an amide bond with citrulline, and a carbonyl group covalently binds to primary amine (C-35 amine) on eribulin. P-aminobenzyloxycarbonyl is self-immolative. Without being bound by theory, it is thought that the self-immolation of pAB involves a spontaneous 1,6-elimination reaction (Jain et al. (2015) Pharm. Res. 32:3526-40). When the linker moiety (for example, Val-Cit) in the ADC is cleaved in a target cell, due to self-immolation of the self-immolative unit (pAB), unmodified eribulin can be released without redundant functional groups remaining on the eribulin side.

Self-immolation chemistry is known in the art and could be readily selected for the ADC according to the present disclosure. In various embodiments, a spacer unit binding the cleavable moiety in the linker to the drug moiety (for example, eribulin) is self-immolative, and undergoes self-immolation concurrently with or immediately before/after cleavage of the cleavable moiety under intracellular conditions.

On the other hand, the maleimide unit (Mal), the oxyethylene unit (PEG), and the propionic acid unit (PA) are arranged between valine and the antibody Ab. α,β-unsaturated carbonyl in maleimide is reactive with a cysteine residue (in particular, a sulfhydryl group (-SH)) in the antibody and functions to bind the linker moiety and eribulin to the antibody. The linker moiety includes the maleimide unit and is linked to the antibody via the maleimide unit. As a result, the drug loading of the antibody (p: the number of eribulin moieties per antibody moiety) can be further improved.

The oxyethylene unit (PEG) is a unit composed of one or more oxyethylene groups and is substantially hydrophilic. Eribulin can be used to reduce the extent to which eribulin may be pumped out of resistant cancer cells through multiple drug resistant (MDR) or functionally similar transporters. In some embodiments, the linker moiety is a shorter PEG linker, and provides improved stability and reduced aggregation over longer PEG linkers. In Formula (I), the number of oxyethylene groups is represented by m, and m is an integer of 1 to 10. m is preferably 2. When m is an integer less than 2, the length of the linker moiety is reduced, and lower aggregation level and/or higher drug loading can be demonstrated as compared to an ADC including a longer linker moiety (for example, m is 8).

In some embodiments, an ADC including a cleavable peptide moiety demonstrates lower aggregation levels, improved antibody:drug ratio, increased on-target killing of cancer cells, decreased off-target killing of non-cancer cells, and/or higher drug loading (p) relative to an ADC including an alternate cleavable moiety. In some embodiments, adding a cleavable moiety increases cytotoxicity and/or potency relative to a non-cleavable linker. In some embodiments, the increased potency and/or cytotoxicity may be observed in a cancer expressing moderate levels of the antigen targeted by the antibody moiety of the ADC (for example, moderate FRA expression). In some embodiments, the cleavable peptide moiety is cleavable by an enzyme, and the linker is an enzyme-cleavable linker. In some embodiments, the enzyme is cathepsin, and the linker is a cathepsin-cleavable linker. In certain embodiments, the enzyme-cleavable linker (for example, the cathepsin-cleavable linker) exhibits one or more of the improved properties mentioned above, as compared to an alternate cleavage mechanism.

In addition, the linker moiety may impact the physico-chemical properties of the ADC. As many cytotoxic agents are hydrophobic in nature, linking them to the antibody with an additional hydrophobic moiety may lead to aggregation. ADC aggregates are insoluble and often limit achievable drug loading onto the antibody, which can negatively affect the potency of the ADC. Protein aggregates of biologies, in general, have also been linked to increased immunogenicity. The linker according to the present embodiment results in ADCs with low aggregation levels and desirable levels of drug loading.

In various embodiments, the linker is designed to facilitate cellular internalization, cleavage after cellular internalization, and bystander killing (the killing of neighboring cells) through and diffusion of the linker-eribulin and/or eribulin alone to neighboring cells. In some embodiments, the linker is designed to minimize cleavage in the extracellular environment and thereby reduce toxicity to off-target tissue (for example, non-cancerous tissue), while preserving ADC binding to target tissue and bystander killing of cancerous tissue that does not express an antigen targeted by the antibody moiety of the ADC, but surrounds target cancer tissue expressing that antigen. The linker moiety according to the present embodiment is particularly effective in providing these functional features, for example, when joining an anti-FRA antibody moiety such as MORAb-003 and a drug moiety such as eribulin. In some embodiments, at least some of these functional features may also be observed without an anti-FRA antibody moiety, and/or without MORAb-003. The linker moiety according to the present embodiment is effective in providing some or all of these functional features, for example, when joining an anti-her2 antibody moiety such as trastuzumab and a drug moiety such as eribulin.

It has been discovered that the ADC demonstrates a particular combination of desirable properties, particularly when paired with an anti-FRA antibody such as MORAb-003 or an antigen-binding fragment thereof. These properties include, but are not limited to, effective levels of drug loading (p ≥ about 4), low aggregation levels, stability under storage conditions or when in circulation in the body (for example, serum stability), retained affinity for target-expressing cells comparable to unconjugated antibody, potent cytotoxicity against target-expressing cells, low levels of off-target cell killing, high levels of bystander killing, and/or effective in vivo anti-cancer activity, all as compared to ADCs using other linker-eribulin and/or antibody moieties.

### 3. Eribulin

The term "eribulin", as used herein, refers to a synthetic analog of halichondrin B, a macrocyclic compound isolated from the marine sponge Halichondria okadais. Eribulin is a microtubule dynamics inhibitor, which is thought to bind tubulin and induce cell cycle arrest at the G2/M phase by inhibiting mitotic spindle assembly. The term "eribulin mesylate" refers to the mesylate salt of eribulin, which is marketed under the trade name Halaven (trade mark). In the ADC according to the present embodiment, eribulin binds to the linker of the ADC via a primary amino group of eribulin.

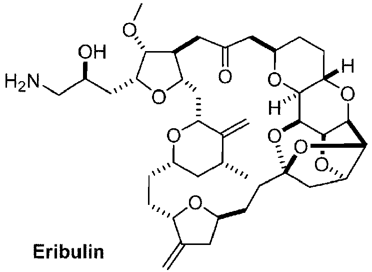

### 4. Antibody-Drug Conjugate (ADC)

In the ADC according to the present embodiment, eribulin binds to the antibody moiety via the above-described linker moiety. The antibody moiety may be, for example, an anti-FRA antibody, an anti-mesothelin antibody, an anti-her2 antibody such as trastuzumab.

The ADC according to the present embodiment can selectively deliver an effective dose of a cytotoxic or cytostatic agent to cancer cells or to tumor tissue. It has been discovered that the ADC has potent cytotoxic and/or cytostatic activity against cells expressing the respective target antigen (for example, FRA or her2). In some embodiments, the cytotoxic and/or cytostatic activity of the ADC is dependent on the target antigen expression level in a cell. In some embodiments, the ADC according to the present disclosure are particularly effective for killing cancer cells expressing a moderate level of target antigen, as compared to cancer cells expressing the same antigen at a low level.

The term "cancer" refers to the physiological condition in mammals in which a population of cells is characterized by unregulated cell growth. Examples of cancers include, but are not limited to, carcinoma, lymphoma, blastoma, sarcoma, and leukemia. More particular examples of such cancers include squamous cell cancer, small cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer (for example, triple negative breast cancer), osteosarcoma, melanoma, colon cancer, colorectal cancer, endometrial (for example, serous) or uterine cancer, salivary gland carcinoma, kidney cancer, liver cancer, prostate cancer, vulvar cancer, thyroid cancer, hepatic carcinoma, and various types of head and neck cancers. Triple negative breast cancer refers to breast cancer that is negative for expression of the genes for estrogen receptor (ER), progesterone receptor (PR), or Her2/neu.

The terms "tumor" refers to any mass of tissue that results from excessive cell growth or proliferation, either benign or malignant, including precancerous lesions.

The terms "cancer cell" and "tumor cell" refer to individual cells or the total population of cells derived from a tumor, including both non-tumorigenic cells and cancer stem cells. As used herein, the term "tumor cell" will be modified by the term "non-tumorigenic" when referring solely to those tumor cells lacking the capacity to renew and differentiate to distinguish those tumor cells from cancer stem cells.

Exemplary high FRA-expressing cancers include but are not limited to ovarian cancer (for example, serous ovarian cancer, clear cell ovarian cancer), lung carcinoid, triple negative breast cancer, endometrial cancer, and non-small cell lung cancer (for example, adenocarcinoma). Exemplary moderate FRA-expressing cancers include but are not limited to gastric cancer and colorectal cancer. Exemplary low FRA-expressing cancers include but are not limited to melanoma and lymphoma. Exemplary high her2-expressing cancers include but are not limited to breast cancer, gastric cancer, esophageal cancer, ovarian cancer, and endometrial cancer. Exemplary moderate her2-expressing cancers include but are not limited to lung cancer and bladder cancer.

The term "inhibit" or "inhibition of', as used herein, means to reduce by a measurable amount, and can include but does not require complete prevention or inhibition.

An "effective amount" of an ADC as disclosed herein is an amount sufficient to perform a specifically stated purpose, for example to produce a therapeutic effect after administration, such as a reduction in tumor growth rate or tumor volume, a reduction in a symptom of cancer, or some other indicia of treatment efficacy. An effective amount can be determined in a routine manner in relation to the stated purpose. The term "therapeutically effective amount" refers to an amount of an ADC effective to treat a disease or disorder in a subject. In the case of cancer, a therapeutically effective amount of ADC can reduce the number of cancer cells, reduce tumor size, inhibit (for example, slow or stop) tumor metastasis, inhibit (for example, slow or stop) tumor growth, and/or relieve one or more symptoms. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

As used herein, "to treat" or "therapeutic" and grammatically related terms, refer to any improvement of any consequence of disease, such as prolonged survival, less morbidity, and/or a lessening of side effects which are the byproducts of an alternative therapeutic modality. As is readily appreciated in the art, full eradication of disease is a preferred but albeit not a requirement for a treatment act. "Treatment" or "treat", as used herein, refers to the administration of a described ADC to a subject, for example, a patient. The treatment can be to cure, heal, alleviate, relieve, alter, remedy, ameliorate, palliate, improve or affect the disorder, the symptoms of the disorder or the predisposition toward the disorder, for example, a cancer.

In some embodiments, a labeled ADC is used. Suitable "labels" include radionuclides, enzymes, substrates, cofactors, inhibitors, fluorescent moieties, chemiluminescent moieties, magnetic particles, and the like.

"Protein", as used herein, is meant at least two covalently bound amino acids. The term encompasses polypeptides, oligopeptides, and peptides. In some embodiments, the two or more covalently bound amino acids are bound via a peptide bond. The protein may be made up of naturally occurring amino acids and peptide bonds, for example when the protein is made recombinantly using expression systems and host cells. Alternatively, the protein may include synthetic amino acids (for example, homophenylalanine, citrulline, ornithine, and norleucine), or peptidomimetic structures, that is, "peptide or protein analogs", such as peptoids. Peptoids are an exemplary class of peptidomimetics whose side chains are appended to the nitrogen atom of the peptide backbone, rather than to the α-carbons (as they are in amino acids), and have different hydrogen bonding and conformational characteristics in comparison to peptides (see, for example, Simon et al. (1992) Proc. Natl. Acad. Sci. USA 89:9367). As such, peptoids can be resistant to proteolysis or other physiological or storage conditions, and effective at permeating cell membranes. Such synthetic amino acids may be incorporated in particular when the antibody is synthesized in vitro by conventional methods well known in the art. In addition, any combination of peptidomimetic, synthetic and naturally occurring residues/structures can be used. "Amino acid" also includes imino acid residues, such as proline and hydroxyproline. The amino acid "R group" or "side chain" may be in either the (L)- or the (S)-configuration. In a specific embodiment, the amino acids are in the (L)- or (S)-configuration.

A "recombinant protein" is a protein made using recombinant techniques using any techniques and methods known in the art, that is, through the expression of a recombinant nucleic acid. Methods and techniques for the production of recombinant proteins are well known in the art.

An "isolated" protein is unaccompanied by at least some of the material with which it is normally associated in its natural state, for example constituting at least about 5%, or at least about 50% by weight of the total protein in a given sample. It is understood that the isolated protein may constitute from 5 to 99.9% by weight of the total protein content depending on the circumstances. For example, the protein may be made at a significantly higher concentration through the use of an inducible promoter or high expression promoter, such that the protein is made at increased concentration levels. The definition includes the production of an antibody in a wide variety of organisms and/or host cells that are known in the art.

For amino acid sequences, sequence identity and/or similarity may be determined using standard techniques known in the art, including, but not limited to, the local sequence identity algorithm of Smith and Waterman (1981) Adv. Appl. Math. 2:482, the sequence identity alignment algorithm of Needleman and Wunsch (1970) J. Mol. Biol. 48:443, the search for similarity method of Pearson and Lipman (1988) Proc. Nat. Acad. Sci. USA 85:2444, computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux et al. (1984) Nucl. Acid Res. 12:387-95, preferably using the default settings, or by inspection. Percent identity may be calculated with reference to "Current Methods in Sequence Comparison and Analysis", Macromolecule Sequencing and Synthesis, Selected Methods and Applications, pp. 127-149 (1988), Alan R. Liss, Inc) or is preferably calculated by Fast DB based upon the following parameters.
Mismatch penalty: 1
Gap penalty: 1
Gap size penalty: 0.33
Joining penalty: 30

An example of a useful algorithm is PILEUP. PILEUP creates a multiple sequence alignment from a group of related sequences using progressive pairwise alignments. It can also plot a tree showing the clustering relationships used to create the alignment. PILEUP uses a simplification of the progressive alignment method of Feng & Doolittle (1987) J. Mol. Evol. 35:351-60; the method is similar to that described by Higgins and Sharp (1989) CABIOS 5:151-3. Useful PILEUP parameters including a default gap weight of 3.00, a default gap length weight of 0.10, and weighted end gaps.

Another example of a useful algorithm is the BLAST algorithm, described in: Altschul et al. (1990) J. Mol. Biol. 215:403-10; Altschul et al. (1997) Nucleic Acids Res. 25:3389-402; and Karin et al. (1993) Proc. Natl. Acad. Sci. USA 90:5873-87. A particularly useful BLAST program is the WU-BLAST-2 program which was obtained from Altschul et al. (1996) Methods in Enzymology 266:460-80. WU-BLAST-2 uses several search parameters, most of which are set to the default values. The adjustable parameters are set with the following values: overlap span=1, overlap fraction=0.125, word threshold (T)=II. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al. (1993) Nucl. Acids Res. 25:3389-402. Gapped BLAST uses BLOSUM-62 substitution scores; threshold T parameter set to 9; the two-hit method to trigger ungapped extensions, charges gap lengths of k a cost of 10+k; Xu set to 16, and Xg set to 40 for database search stage and to 67 for the output stage of the algorithms. Gapped alignments are triggered by a score corresponding to about 22 bits.

Generally, the amino acid homology, similarity, or identity between proteins disclosed herein and variants thereof, including variants of FRA, variants of her2, variants of tubulin sequences, and variants of antibody variable domains (including individual variant CDRs), are at least 80% to the sequences described herein, and more typically with preferably increasing homologies or identities of at least 85%, 90%, 91%, 92%, 93%, 94%, 95%, 96%, 97%, 98%, 99%, and almost 100% or 100%.

In a similar manner, "percent (%) nucleic acid sequence identity" with respect to the nucleic acid sequence of the antibodies and other proteins identified herein is defined as the percentage of nucleotide residues in a candidate sequence that are identical to the nucleotide residues in the coding sequence of the antigen binding protein. A specific method utilizes the BLASTN module of WU-BLAST-2 set to the default parameters, with overlap span and overlap fraction set to 1 and 0.125, respectively.

While the site or region for introducing an amino acid sequence variation is predetermined, the mutation per se need not be predetermined. For example, in order to optimize the performance of a mutation at a given site, random mutagenesis may be conducted at the target codon or region and the expressed antigen binding protein CDR variants screened for the optimal combination of desired activity. Techniques for making substitution mutations at predetermined sites in DNA having a known sequence are well known, for example, MI3 primer mutagenesis and PCR mutagenesis.

Next, a method of producing the ADC represented by Formula (I) according to an embodiment of the present invention will be described.

The present embodiment is the method of producing the antibody-drug conjugate (ADC) represented by Formula (I) and includes the following steps 1 and 2. 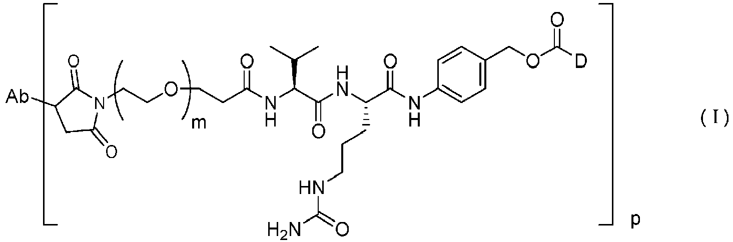 [In the formula,
Ab is an antibody or an antigen-binding fragment thereof,
D is eribulin,
m is an integer of 1 to 10, and
p is an integer of 1 to 8.]

Step 1: a step of obtaining a compound represented by Formula (B) by reaction of eribulin or a salt thereof with a compound represented by Formula (A).

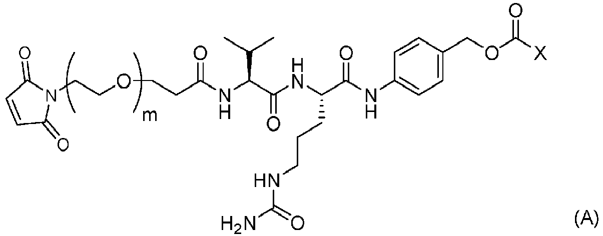

[In the formula, m is an integer of 1 to 10 and X is a phenoxy group or a nitrophenoxy group.]

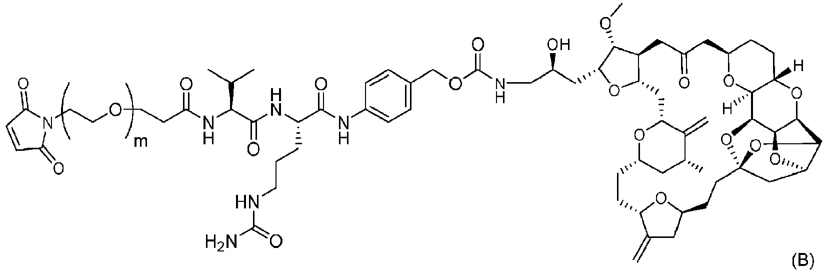

[In the formula, m is an integer of 1 to 10.]

Step 2: a step of obtaining the antibody-drug conjugate represented by Formula (I) by reaction of the compound represented by Formula (B) with Ab.

The step 1 is a step of obtaining a compound represented by Formula (B) by reaction of eribulin or a salt thereof with a compound represented by Formula (A).

Eribulin may be a free form of eribulin or may be a salt of eribulin. Examples of the salt of eribulin include eribulin mesylate. The amount of eribulin or the salt thereof used may be 1 to 1000 g or may be 10 to 300 g in terms of the free form of eribulin.

The compound represented by Formula (A) is a compound corresponding to the linker moiety in the ADC, is reactive with the antibody in the terminal maleimide structure, and is reactive with eribulin at the terminal X on the opposite side. X is a phenoxy group or a nitrophenoxy group. The nitrophenoxy group may be any one of an o-nitrophenoxy group, an m-nitrophenoxy group, and a p-nitrophenoxy group. When X is a phenoxy group or a nitrophenoxy group, X is reactive with a primary amino group at the terminal of eribulin. The compound represented by Formula (A) can be prepared using a method described below.

The amount of the compound represented by Formula (A) used may be 0.5 to 2.0 mol, 0.6 to 3.0 mol, 1.0 to 2.0 mol, or 1.3 to 1.8 mol with respect to 1 mol of eribulin.

In the step 1, a base may be used. The base is not limited as long as it does not inhibit the reaction of eribulin and the compound represented by Formula (A), and examples of the base include a tertiary amine such as triethylamine or N,N-diisopropylethylamine and a nitrogen-containing aromatic compound such as pyridine or 2,6-lutidine. The amount of the base used may be 0.5 to 3.0 mol, 1.0 to 2.0 mol, or 1.1 to 1.5 mol with respect to 1 mol of eribulin.

The step 1 can be performed in the absence of a solvent or in a solvent and is preferably performed in a solvent. The solvent is not limited as long as it does not inhibit the reaction of eribulin and the compound represented by Formula (A), and examples of the solvent include N,N-dimethylformamide (DMF), dimethyl sulfoxide (DMSO), N-methylpyrrolidone (NMP), and pyridine. The amount of the solvent used may be 5 to 20 mL or 6 to 15 mL with respect to 1 g of eribulin.

The reaction temperature in the step 1 is not limited as long as it is a temperature at which the reaction of eribulin and the compound represented by Formula (A) progresses. The reaction temperature may be normal temperature or 20°C to 25°C.

In order to accelerate the reaction of the step 1, an accelerator such as 4-dimethylaminopyridine may be used. The amount of the accelerator used may be 0.01 to 0.8 g or 0.1 to 0.3 g with respect to 1 mol of eribulin.

Hereinafter, an example of a purification method will be described. However, the purification method is not limited to this example, and purification may be performed using a method known in the chemistry. When the reaction of the step 1 is finished, the reaction mixture can be purified on a reverse phase column. The reverse phase column is, for example, a column in which silica gel having an octadecyl group (C18) is used as a filler. The reaction mixture is placed on the top of the reverse phase column, and is extracted with a polar solvent such as acetonitrile or water. Optionally, it is preferable that acetic acid or the like is used such that the pH of the extraction solvent is acidic. Methylene chloride is added to the obtained extract, and the formed compound represented by Formula (B) is transported to a methylene chloride layer (organic layer). During the extraction, optionally, the pH may be adjusted with a sodium bicarbonate aqueous solution. The obtained organic layer may be neutralized with a residual acid such as a sodium bicarbonate aqueous solution. The obtained organic layer is concentrated under reduced pressure to dissolve the residue in methylene chloride, methanol, or the like, and the solution is added dropwise to pentane. As a result, the compound represented by Formula (B) is precipitated. The obtained solid is washed with pentane and is dried under reduced pressure. As a result, the desired compound can be obtained.

The step 2 is a step of obtaining the antibody-drug conjugate represented by Formula (I) by reaction of the compound represented by Formula (B) with Ab. The cysteine residue in the antibody moiety (Ab) undergoes Michael addition to the maleimide structure in the compound represented by Formula (B) such that a covalent bond is formed in between. A plurality of the compounds represented by Formula (B) can bind to one antibody moiety Ab. The number of formed bonds corresponds to p in Formula (A).

The amount of the compound represented by Formula (B) used may be 1.0 to 8.0 mol or 3.0 to 5.0 mol with respect to the antibody moiety Ab.

Typically, the step 2 is performed in a buffer solution. The buffer solution is not limited as long as it does not modify the antibody Ab to be used and does not inhibit the binding to the compound represented by Formula (B). The buffer solution may be a phosphate buffer solution, a borate buffer solution, a tris(hydroxymethyl)aminomethane buffer solution, an ethylenediaminetetraacetic acid buffer solution, or any combination thereof. The pH of the buffer solution is preferably 6.0 to 8.0 and more preferably 6.5 to 7.5.

In the step 2, a polar organic solvent may be added to the buffer solution. The polar organic solvent is not limited as long as it can be mixed with the buffer solution, and can improve the solubility of the compound represented by Formula (B). For example, a solution in which the compound represented by Formula (B) is dissolved in the polar organic solvent may be added to the buffer solution in which the antibody Ab is dissolved.

Examples of the polar organic solvent include N,N-dimethylformamide, N,N-dimethylacetamide, and dimethyl sulfoxide. The amount of the organic solvent used may be 60 to 300 mL, 110 to 240 mL, 130 to 210 mL, or 140 to 180 mL with respect to 1 mg of the compound represented by Formula (B).

The reaction temperature in the step 2 is not limited as long as it is a temperature at which the reaction of the compound represented by Formula (B) and the antibody Ab progresses. The reaction temperature may be normal temperature or 20°C to 25°C.

More specifically, the reaction temperature is as follows. First, the antibody Ab is filtered through a filter (permeable membrane) and dissolved by adding the buffer solution thereto. By adding TCLP-HCl (tris(2-carboxyethyl)phosphine hydrochloride) to the obtained solution for partial reduction, a disulfide bond in the molecule of the antibody is cleaved to form a sulfhydryl group. Next, the compound represented by Formula (B) or the solution in which the compound represented by Formula (B) is dissolved in the polar organic solvent is added such that the compound represented by Formula (B) and the antibody bind to each other. When the reaction of the step 2 is finished, for example, N-acetylcysteine can be added. N-acetylcysteine is caused to react with the remaining compound represented by Formula (B) and is also caused to react with the sulfhydryl group formed by the partial reduction.

Hereinafter, an example of a purification method will be described. However, the purification method is not limited to this example, and purification may be performed using a method known in the chemistry. After finishing the reaction, the buffer solution of the reaction solution is replaced with another buffer solution using a tangential flow filtration method (TFF). Next, a citric acid aqueous solution is added, is filtered through a filter, and is purified with the buffer solution.

Here, a method of producing the compound represented by Formula (A) will be described. The compound represented by Formula (A) can be produced, for example, from a compound represented by Formula (1) and a compound represented by Formula (2) through two steps as shown below.

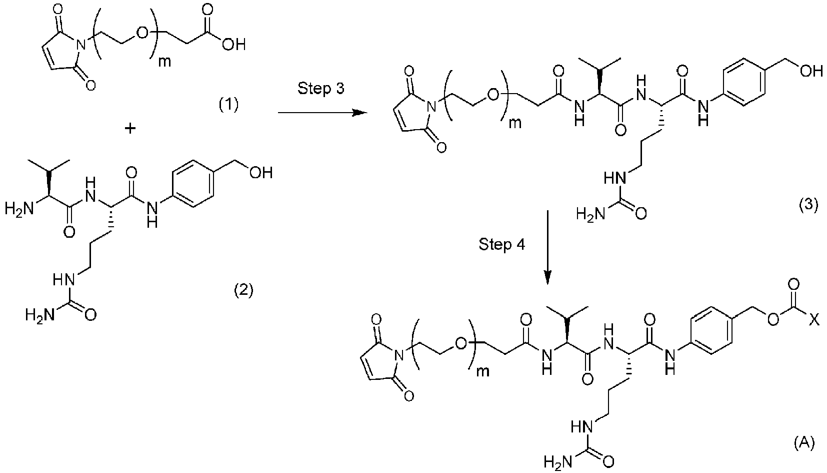

A step 3 is a step of activating a carboxy group of the compound represented by Formula (1) and condensing the compound represented by Formula (1) with the compound represented by Formula (2). A compound represented by Formula (3) is obtained by condensation of the compound represented by Formula (1) and the compound represented by Formula (2).

The compound represented by Formula (1) is available from Tokyo Chemical Industry Co., Ltd. as a trade name of "Mal-PEG₂-acid" (catalog code: M3203). In addition, the compound represented by Formula (1) may be produced with a method known in the organic chemistry using tert-butyl acrylate and the like with polyethylene glycol having a corresponding length as a starting material.

The amount of the compound represented by Formula (1) used may be 1.0 to 1.5 mol or 1.1 to 2.0 mol with respect to 1 mol of the compound represented by Formula (2).

The compound represented by Formula (2) is available from Tokyo Chemical Industry Co., Ltd. as a trade name of "Fmoc-Val-Cit-PAB-OH" (catalog code: F1223). In addition, the compound represented by Formula (2) may be produced with a method known in the organic chemistry using N-Fmoc-citrulline, p-aminobenzyl alcohol, and N-Fmoc-valine. "Fmoc" is one protective group for the amino group and is a 9-fluorenylmethyloxycarbonyl group.

It is preferable that the step 3 is performed in the presence of a condensing agent. The condensing agent is not limited as long as it can accelerate the condensation reaction of carboxylic acid and primary amine, and examples of the condensing agent include dicyclohexylcarbodiimide (DCC), 1-(3-dimethylaminopropyl)-3-ethylcarbodiimide, and 4-(4,6-dimethoxy-1,3,5-triazin-2-yl)-4-methylmorpholinium chloride (DMT-MM).

The amount of the condensing agent used may be 1.0 to 2.0 mol or 1.3 to 1.5 mol with respect to 1 mol of the compound represented by Formula (1).

In the step 3, a base may be used. The base is not limited as long as it does not inhibit the reaction of the compound represented by Formula (1) and the compound represented by Formula (2), and examples of the base include a tertiary amine such as triethylamine or N,N-diisopropylethylamine and a nitrogen-containing aromatic compound such as pyridine or 2,6-lutidine. The amount of the base used may be 0.5 to 2.0 mol, 1.0 to 1.5 mol, or 1.1 to 1.3 mol with respect to 1 mol of the compound represented by Formula (1).

The step 3 can be performed in the absence of a solvent or in a solvent and is preferably performed in a solvent. The solvent is not limited as long as it does not inhibit the reaction of the compound represented by Formula (1) and the compound represented by Formula (2), and examples of the solvent include N,N-dimethylformamide (DMF), tetrahydrofuran (THF), methanol (MeOH), and a mixed solvent thereof. The amount of the solvent used may be 10 to 40 mL or 20 to 30 mL with respect to 1 g of the compound represented by Formula (1).

The reaction temperature in the step 3 is not limited as long as it is a temperature at which the reaction of the compound represented by Formula (1) and the compound represented by Formula (2) progresses. The reaction temperature may be normal temperature or 15°C to 35°C.

A step 4 is a step of obtaining the compound represented by Formula (A) by acylating a hydroxyl group in the compound represented by Formula (3) using an acylating agent.

The amount of the compound represented by Formula (3) used can be appropriately selected depending on the reaction. For example, the amount of the compound represented by Formula (3) used may be 10 to 500 g or 100 to 300 g.

Examples of the acylating agent include diphenyl carbonate, bis(4-nitrophenyl) carbonate, bis(2-nitrophenyl) carbonate, bis(3-nitrophenyl) carbonate, phenyl chloroformate, 4-nitrophenyl chloroformate, 2-nitrophenyl chloroformate, and 3-nitrophenyl chloroformate. A part of the acylating agent corresponds to X in Formula (A), and the kind of the acylating agent can be selected depending on the selection of the desired X group.

The amount of the acylating agent used may be 1.0 to 5.0 mol or 2.0 to 4.0 mol with respect to 1 mol of the compound represented by Formula (3).

The step 4 can be performed in the absence of a solvent or in a solvent and is preferably performed in a solvent. The solvent is not limited as long as it does not inhibit the acylation reaction of the compound represented by Formula (3), and examples of the solvent include N,N-dimethylformamide (DMF), N,N-dimethylacetamide, toluene, dimethyl sulfoxide (DMSO), and N,N-dimethylacetamide (DMA). The amount of the solvent used may be 5 to 20 mL or 6 to 15 mL with respect to 1 g of the compound represented by Formula (3).

The reaction temperature in the step 4 is not limited as long as it is a temperature at which the acylation reaction of the compound represented by Formula (3) progresses. The reaction temperature may be normal temperature or 20°C to 25°C.

### Examples

Hereinafter, the present invention will be described in more detail using Production Examples and Comparative Production Examples.

Abbreviations used in Examples are used are they are in the art, and, for example, are as follows.
DIPEA: N,N-diisopropylethylamine
DMA: N,N-dimethylacetamide
DMF: N,N-dimethylformamide
DMT-MM: 4-(4,6-dimethoxy-1,3,5-triazin-2-yl)-4-methylmorpholinium chloride
EDTA: ethylenediaminetetraacetic acid
THF: tetrahydrofuran

### <Production Example 1>

(11S,14S)-14-[3-(carbamoylamino)propyl]-1-(2,5-dioxo-2,5-dihydro-1H-pyrr ole-1-yl)-N-[4-(hydroxymethyl)phenyl]-9,12-dioxo-11-(propan-2-yl)-3,6-dioxa-10,13 -diazapentadecane-15-amide

A mixture of (2S)-2-{[(2S)-2-amino-3-methylbutanoyl]amino}-5-(carbamoylamino)-N-[4-(hydrox ymethyl)phenyl]pentanamide (184 g), 3-{2-[2-(2,5-dioxo-2,5-dihydro-1H-pyrrol-1-yl)ethoxy]ethoxy}propanoic acid (150 g), DMT-MM (174 g), methanol (2.76 L), and THF (2.76 L) was stirred in a nitrogen atmosphere at 15°C to 35°C for 24 hours. The reaction mixture was concentrated under reduced pressure, and acetonitrile was added thereto. The precipitated precipitate was filtered and was further washed with acetonitrile. The obtained solid was mixed with acetonitrile and was filtered to obtain a solid. The obtained solid was dried under reduced pressure to obtain a title compound (267 g, 89% yield).

### <Production Example 2>

(4-{[(11S,14S)-14-[3-(carbamoylamino)propyl]-1-(2,5-dioxo-2,5-dihydro-1H -pyrrole-1-yl)-9,12,15-trioxo-11-(propan-2-yl)-3,6-dioxa-10,13-diazapentadecane-15-yl]amino}phenyl)methyl=4-nitrophenyl=carbonate

A mixture of the compound obtained in Production Example 1 (250 g), bis(4-nitrophenyl)carbonate (615 g), DIPEA (157 g), and DMF (5.0 L) was stirred in a nitrogen atmosphere at 15°C to 35°C for 3 hours. The reaction mixture was concentrated under reduced pressure and was purified on a silica gel column (methylene chloride/acetone), and a fraction including the title compound was concentrated under reduced pressure. As a result, the title compound (162 g, 51% yield) was obtained as a solid.

### <Production Example 3>

(4-{[(11S,14S)-14-[3-(carbamoylamino)propyl]-1-(2,5-dioxo-2,5-dihydro-1H -pyrrole-1-yl)-9,12,15-trioxo-11-(propan-2-yl)-3,6-dioxa-10,13-diazapentadecane-15-yl]amino}phenyl)methyl={(2S)-2-hydroxy-3-[(2R,3R,3aS,7R,8aS,9S,10aR,HS,12R, 13aR,13bS,15S,18S,21S,24S,26R,28R,29aS)-3-methoxy-26-methyl-20,27-dimethyli denehexacosahydro-11,15:18,21:24,28-triepoxy-7,9-ethano-12,15-methano-9H,15H-f uro[3,2-i]furo[2',3':5,6]pyrano[4,3-b][1,4]dioxacyclopentacosin-5(4H)-one-2-yl]propy l}carbamate

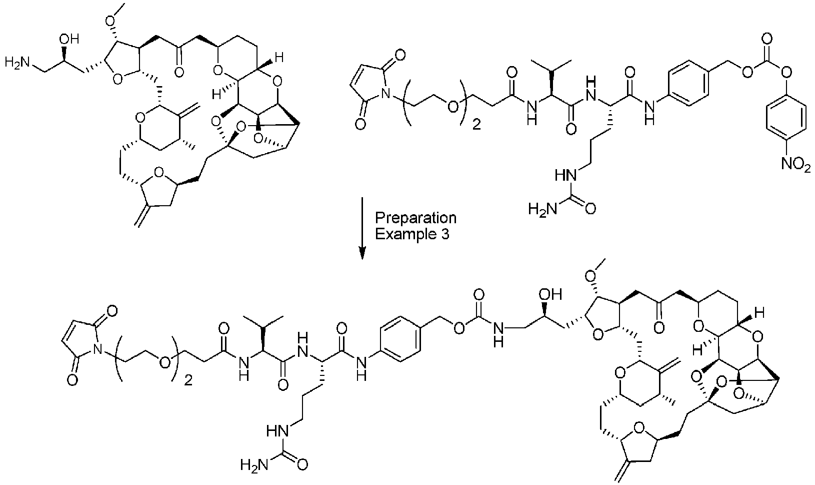

A mixture of eribulin mesylate (93 g), DIPEA (26 mL), the compound obtained in Production Example 2 (116 g), and DMF (930 mL) was stirred in a nitrogen atmosphere at 20°C to 25°C for 20 hours. The reaction mixture was purified on a reverse phase column (trade name; Kromasil C18) (acetonitrile/water/acetic acid), and a fraction including a title compound was extracted with methylene chloride. The organic layer was sequentially washed with a sodium bicarbonate aqueous solution and water and subsequently was concentrated under reduced pressure. The concentrated residue was dissolved in a mixed solvent of methylene chloride, methanol, and acetic acid, and the solution was added dropwise to pentane. The precipitated precipitate was filtered, and the obtained solid was washed with pentane and dried under reduced pressure. As a result, a title compound (109.6 g, 71% yield) was obtained.

¹H-NMR(400 MHz, CD₃OD): δ (ppm) 7.59 (d, J = 8.4 Hz, 2H), 7.31 (d, J = 8.4 Hz, 2H), 6.81 (s, 2H), 5.13 (s, 1H), 5.06 (d, J = 12.4 Hz, 1H), 5.02 (s, 1H), 5.01 (d, J = 12.4 Hz, 1H), 4.87 (s, 1H), 4.82 (s, 1H), 4.71 (t, J = 4.0 Hz, 1H), 4.61 (t, J = 4.4 Hz, 1H), 4.50 (dd, J = 5.2, 9.2 Hz, 1H), 4.47 (d, J = 10.8 Hz, 1H), 4.32-4.27 (m,2H), 4.19 (dd, J = 6.8, 11.6 Hz, 1H), 4.13-4.07 (m, 2H), 3.98 (t, J = 10.4 Hz,1H), 3.88-3.82 (m, 3H), 3.76-3.64 (m, 6H), 3.62-3.51 (m, 6H), 3.38 (s, 3H), 3.22-3.08 (m, 4H), 2.93 (dd, J = 2.4, 9.6 Hz, 1H), 2.92-2.84 (m, 1H), 2.76-2.63 (m, 2H), 2.52(t, J = 6.0 Hz,2H), 2.44-2.29 (m, 5H), 2.21-1.97 (m, 8H), 1.93-1.83 (m, 3H), 1.80-1.66 (m, 5H), 1.66-1.28 (m, 10H), 1.11 (d, J = 6.4 Hz, 3H), 1.07-1.01 (m, 1H), 0.99 (d, J = 6.8 Hz, 3H), 0.97 (d, J = 6.4 Hz, 3H).

LCMS (M+H): m/z 1374.9

### <Comparative Production Example 1>

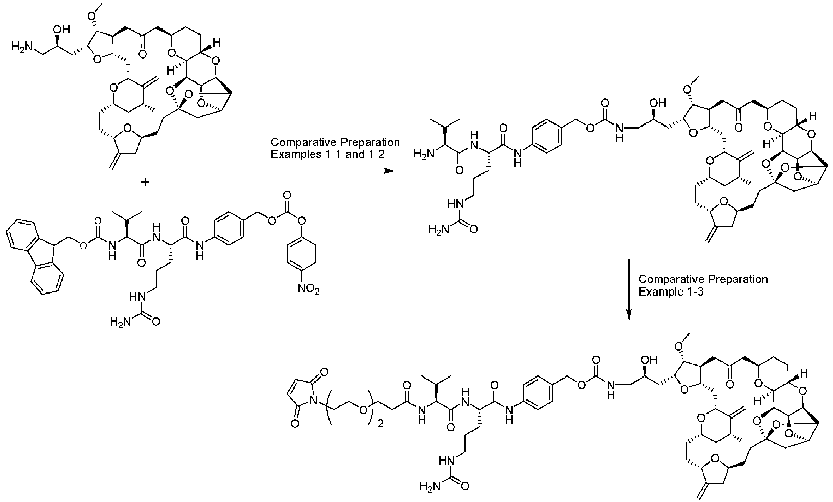

### <Comparative Production Example 1-1>

4-{[(2S)-5-(carbamoylamino)-2-{[(2S)-2-{[(9H-fluorene-9-ylmethoxy)carbo nyl]amino}3-methylbutanoyl]amino}pentanoyl]amino}benzyl={(2S)-2-hydroxy-3-[( 2R,3R,3aS,7R,8aS,9S,10aR,11S,12R,13aR,13bS,15S,18S,21S,24S,26R,28R,29aS)-3 -methoxy-26-methyl-20,27-dimethylidenehexacosahydro-11,15:18,21:24,28-triepoxy -7,9-ethano-12,15-methano-9H,15H-furo[3,2-i]furo[2',3':5,6]pyrano[4,3-b][1,4]dioxa cyclopentacosin-5(4H)-one-2-yl]propyl}carbamate

A mixture of eribulin mesylate (93 g), DIPEA (25 mL), 4-{[(2S)-5-(carbamoylamino)-2-{[(2S)-2-{[(9H-Suorene-9-ylmethoxy)carbonyl]ami no}3-methylbutanoyl]amino}pentanoyl]amino}benzyl=4-nitrophenyl carbonate (103.6 g), and DMF (930 mL) was stirred in a nitrogen atmosphere at 20°C to 25°C for 16 hours.

### <Comparative Production Example 1-2>

4-{[(2S)-2-{[(2S)-2-amino-3-methylbutanoyl]amino}-5-(carbamoylamino)pe ntanoyl]amino}benzyl={(2S)-2-hydroxy-3-[(2R,3R,3aS,7R,8aS,9S,10aR,HS,12R,13 aR,13bS,15S,18S,21S,24S,26R,28R,29aS)-3-methoxy-26-methyl-20,27-dimethylide nehexacosahydro-11,15:18,21:24,28-triepoxy-7,9-ethano-12,15-methano-9H,15H-fur o[3,2-i]furo[2',3':5,6]pyrano[4,3-b][1,4]dioxacyclopentacosim-5(4H)-one-2-yl]propyl} carbamate

Diethylamine (234 mL) was added to the reaction mixture and was stirred at 20°C to 25°C for 0.5 hours. Ethyl acetate and hydrochloric acid were added to the reaction mixture, and the aqueous layer was washed with heptane. Methyltetrahydrofuran and a potassium carbonate sodium chloride aqueous solution were added to the aqueous layer, and the organic layer was washed with a sodium chloride aqueous solution. The organic layer was dried with magnesium sulfate and concentrated under reduced pressure.

¹H-NMR (400 MHz, CD₃OD): δ (ppm) 7.56 (d, J = 8.4 Hz, 2H), 7.32 (d, J = 8.4 Hz, 2H), 5.14 (s, 1H), 5.06 (d, J = 12.4 Hz, 1H), 5.03 (s, 1H), 5.01 (d, J = 12.4 Hz,1H), 4.87 (s, 1H), 4.83 (s, 1H), 4.71 (t, J = 4.4 Hz, 1H), 4.62 (t, J = 4.4 Hz, 1H), 4.57 (dd, J = 4.8, 8.8 Hz, 1H), 4.47 (d, J = 10.8 Hz, 1H), 4.32-4.27 (m, 2H), 4.18 (dd, J = 4.8, 6.4 Hz, 1H), 4.13-4.07 (m, 2H), 3.98 (t, J = 10.4 Hz, 1H), 3.88-3.82 (m, 3H), 3.76-3.70 (m, 4H), 3.60 (d, J = 6.0 Hz, 1H), 3.38 (s, 3H), 3.26-3.10 (m, 3H), 2.93 (dd, J =2.0, 11.2 Hz, 1H), 2.91-2.84 (m, 1H), 2.75-2.64 (m, 2H), 2.44-2.29 (m, 5H), 2.21-1.97 (m, 8H), 1.93-1.83 (m, 3H), 1.79-1.72 (m, 5H), 1.68-1.29 (m, 8H), 1.11 (d, J = 6.8 Hz, 3H), 1.07-1.01 (m, 1H), 1.06 (d, J = 7.2 Hz, 3H), 1.02 (d, J = 7.2 Hz, 3H)

LCMS(M+H): m/z1135.7

### <Comparative Production Example 1-3>

(4-{[(11S,14S)-14-[3-(carbamoylamino)propyl]-1-(2,5-dioxo-2,5-dihydro-1H -pyrrole-1-yl)-9,12,15-trioxo-11-(propan-2-yl)-3,6-dioxa-10,13-diazapentadecane-15-yl]amino}phenyl)methyl={(2S)-2-hydroxy-3-[(2R,3R,3aS,7R,8aS,9S,10aR,HS,12R, 13aR,13bS,15S,18S,21S,24S,26R,28R,29aS)-3-methoxy-26-methyl-20,27-dimethyli denehexacosahydro-11,15:18,21:24,28-triepoxy-7,9-ethano-12,15-methano-9H,15H-f uro[3,2-i]furo[2',3':5,6]pyrano[4,3-b][1,4]dioxacyclopentacosin-5(4H)-one-2-yl]propy l}carbamate

41-[2-(2-{3-[(2,5-dioxopyrrolidin-1-yl)oxy]-3-oxopropoxy}ethoxy)ethyl]-1H -pyrrole-2,5-dione (47.9 g), DIPEA (20 mL), and DMF (913 mL) were added to the concentrated residue obtained in Comparative Production Example 1-2 and were stirred at 20°C to 25°C for 3 hours.

The reaction mixture was purified on a reverse phase column (trade name; SILMERCK 10013) (acetonitrile/water/acetic acid), and a fraction including a title compound was extracted with methylene chloride. The organic layer was washed with a sodium bicarbonate aqueous solution and water and subsequently was concentrated under reduced pressure. The concentrated residue was dissolved in methylene chloride, methanol, and acetic acid, and the solution was added dropwise to pentane. As a result, the precipitated precipitate was filtered. The obtained solid was washed with pentane and dried under reduced pressure. As a result, a title compound (88.1 g, 57% yield in three steps) was obtained.

### <Production Example 4>

1493 mL of a buffer aqueous solution for dilution (10 mmol/L of trisodium phosphate, 100 mmol/L of sodium chloride, 3 w/v% sucrose, and pH 6.5) and 1098 mL of a buffer solution for pH adjustment (0.25 mol/L of tris(hydroxymethyl)aminomethane hydrochloride, 20 mmol/L of EDTA, pH 7.7) were added to 7854 mL of a MORAb-003-containing buffer aqueous solution (26.6 mg/mL of MORAb-003, 10 mmol/L of trisodium phosphate, 100 mmol/L of sodium chloride, 3 w/v% sucrose, and pH 6.5). A tris(2-carboxyethyl)phosphine aqueous solution (10 mmol/L, 311 g) was added at 20°C and stirred for 2.5 hours.

A DMA solution of the compound obtained in Production Example 3 (8.37 mmol/L, 809 mL) was added and stirred for 0.5 hours. An N-acetylcysteine aqueous solution (30 mmol/L, 452 g) was added to stop the reaction and was stirred for 0.5 hours. The antibody-drug conjugate (ADC) was purified by diafiltration under the following conditions.

### (Purification Conditions)

Filter (permeable membrane): Pellicon 3 (1.14 m² 30 kDa x1, 0.57 m² 30 kDa x1)
TMP (transmembrane pressure difference): 15 psi
Additional buffer solution: 25 mmol/L of citric acid aqueous solution (pH 6.3)
Temperature: 17.0°C to 23.0°C
Permeate: 163.2 kg

By adding 25 mmol/L of a citric acid aqueous solution (3013 g, pH 6.3) and 5668 g of a buffer aqueous solution for conditioning (25 mmol/L of citric acid, 1 mol/L of sucrose, 0.24 w/v%, Polysorbate 80, pH 6.3), an aqueous solution of ADC (protein concentration: 10.6 mg/mL, total weight: 20.9 kg, content of ADC: 213.430 g, 99% yield) was obtained. Next, the aqueous solution of ADC was sterile filtered (Millipore Durapore PVDF).

## Claims

1. A method of producing an antibody-drug conjugate represented by Formula (I), 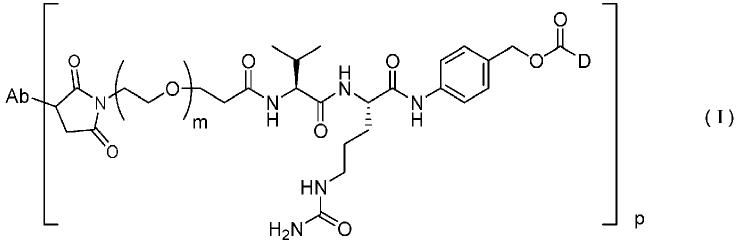
in the formula, Ab is an antibody or an antigen-binding fragment thereof, D is eribulin, m is an integer of 1 to 10, and p is an integer of 1 to 8,
the method comprising:
a step 1 of obtaining a compound represented by Formula (B) by reaction of eribulin or a salt thereof with a compound represented by Formula (A), 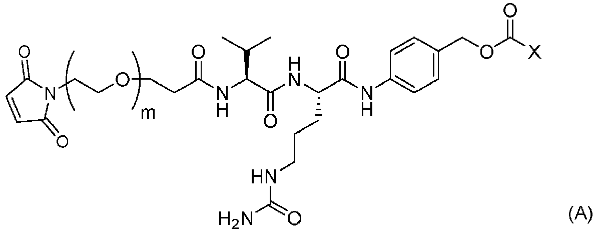
in the formula, m is an integer of 1 to 10 and X is a phenoxy group or a nitrophenoxy group, and 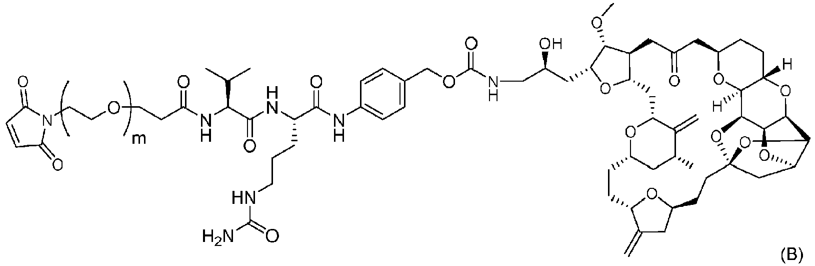
in the formula, m is an integer of 1 to 10; and
a step 2 of obtaining the antibody-drug conjugate represented by Formula (I) by reaction of the compound represented by Formula (B) with Ab.

2. The method according to claim 1,
wherein Ab comprises (i) a heavy chain domain comprising an amino acid sequence represented by SEQ ID NO: 1 and a light chain domain comprising an amino acid sequence represented by SEQ ID NO: 6, (ii) a heavy chain variable domain comprising an amino acid sequence represented by SEQ ID NO: 23 and a light chain variable domain comprising an amino acid sequence represented by SEQ ID NO: 24, (iii) a heavy chain variable domain comprising an amino acid sequence represented by SEQ ID NO: 27 and a light chain variable domain comprising an amino acid sequence represented by SEQ ID NO: 28, or (iv) a heavy chain domain comprising an amino acid sequence represented by SEQ ID NO: 347 and a light chain domain comprising an amino acid sequence represented by SEQ ID NO: 308.

3. The method according to claim 1 or 2, wherein p is 3 or 4.

4. The method according to any one of claims 1 to 3, wherein eribulin or the salt thereof is eribulin methanesulfonate.

5. The method according to any one of claims 1 to 4, wherein the step 1 is performed in the presence of a base.

6. A method of producing a compound represented by Formula (B), 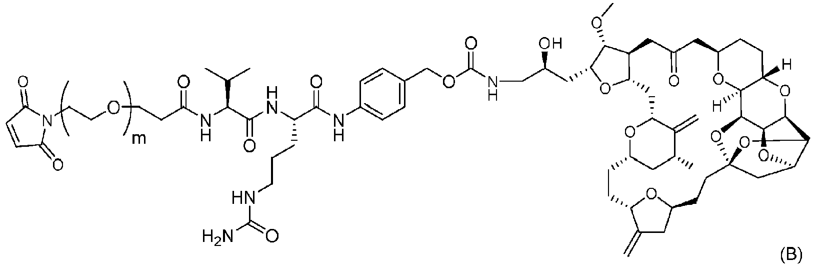
in the formula, m is an integer of 1 to 10,
the method comprising:
a step of obtaining a compound represented by Formula (B) by reaction of eribulin or a salt thereof with a compound represented by Formula (A), 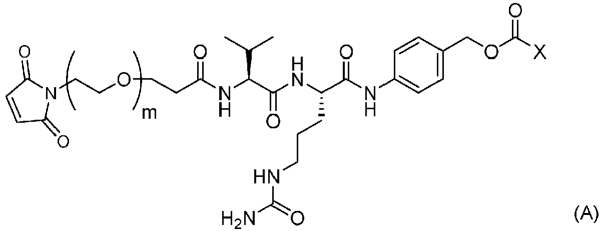
in the formula, m is an integer of 1 to 10 and X is a phenoxy group or a nitrophenoxy group.

7. The method according to claim 6, wherein the step is performed in the presence of a base.

8. A compound represented by Formula (A), 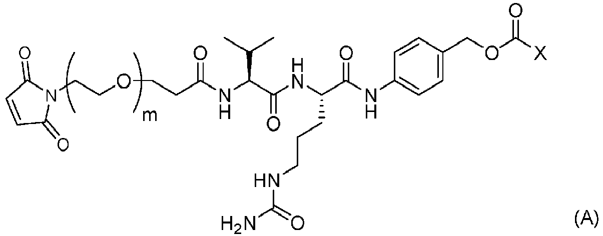 in the formula, m is an integer of 1 to 10 and X is a phenoxy group or a nitrophenoxy group.
